# EUROPEAN PATENT APPLICATION

(11) **EP 3 992 297 A1**
(43) Date of publication of application: **04.05.2022**
(21) Application number: 20204175.2
(22) Date of filing: 27.10.2020
(51) Int. Cl.: C12N 15/82, C12Q 1/6895

(54) **METHOD FOR SELECTING OR IDENTIFYING A BRASSICA NAPUS PLANT HAVING RESISTANCE TO FUNGAL PATHOGEN**

(71) Applicant: KWS SAAT SE & Co. KGaA, 37574 Einbeck (DE)
(72) Inventor: BREUER, Frank, 37574 Einbeck (DE); KLOIBER-MAITZ, Monika, 37574 Einbeck (DE); OUZUNOVA, Milena, 37077 Göttingen (DE); GERTZ, Andreas, 37574 Einbeck (DE)

(57) **Abstract**

The present invention relates to a method for selecting or identifying a *B*. *napus* plant having resistance to Phoma stem canker, such as resistance to fungal pathogen(s) *L. maculans and*/*or L. biglobosa,* comprising detecting in said *B*. *napus* plant, or part thereof (such as tissue or seed), the presence or absence of at least one marker which is on a chromosomal interval between SEQ ID No: 1 and SEQ ID No: 10. The present invention also provides kits of primers for identifying a *B*. *napus* plant having resistance to Phoma stem canker, such as resistance to fungal pathogen(s) *L. maculans* and/or *L. biglobosa.* Also provided is the use of a marker as defined herein, or a sequence selected from SEQ ID No: 1-10, or a kit of primers according to the present invention to select a *B*. *napus* plant having resistance to Phoma stem canker, such as resistance to fungal pathogen(s) *L. maculans and*/*or L. biglobosa.*

SEQUENCE LISTING

<110>  KWS SAAT SE & CO. KGAA

<120>  METHOD

<130>  G204572

<160>  40

<170>  PatentIn version 3.5

<210>  1
<211>  101
<212>  DNA
<213>  Artificial Sequence

<220>
<223>  marker ra24982s01

<400>  1
cgtaccgatt gagactgaaa catccttata tgcatccgag tgtccattct yaggcctcaa      60

agataagcac tgaccaaata ctcctctgaa agcttaactc t                         101

<210>  2
<211>  101
<212>  DNA
<213>  Artificial Sequence

<220>
<223>  marker ra74607s01

<400>  2
ttggtgttac taatttttkc tgagcagtcg gtaattagtc gctaataagt yggccatttt      60

tctataaaat ctaagcgata tcttccatac atttctgctc a                         101

<210>  3
<211>  101
<212>  DNA
<213>  Artificial Sequence

<220>
<223>  marker ra74625s01

<400>  3
tatttttgtg gaatcttccc taaaagaatt acagtgtaaa tttgtagtat satcaataca      60

ttggatattt tgatwtaatc aaaaacacta ggtgataacc c                         101

<210>  4
<211>  101
<212>  DNA
<213>  Artificial Sequence

<220>
<223>  marker ra74605s01

<400>  4

tcyacattts ggcaagacta tccacctgaa tgttttagag agcttgcttt ygaagtggta 60

actwaccata ctccttttag tcttaatktt ttgggttggt g 101

<210> 5
<211> 101
<212> DNA
<213> Artificial Sequence

<220>
<223> marker ra74601s01

<400> 5
tggtctccat tgacgtaagg agatcaagaa aaagctatga gtctaaattg yctatcatgt 60

caagctcttt cacgaaccga ttcaaacaaa gaatttgatc t 101

<210> 6
<211> 101
<212> DNA
<213> Artificial Sequence

<220>
<223> marker ra74589s02

<400> 6
gtagagatat gtggccttca aagtgtcact caatttctgg ccattgatga ytaaaacaag 60

taaacaactt catagttttc ttatgccagt tttatgatat g 101

<210> 7
<211> 101
<212> DNA
<213> Artificial Sequence

<220>
<223> marker ra74593s01

<400> 7
aacgaaaaca maattttata gaggtagaaa atgaaatcac ttatggagag ycaatagtaa 60

acagatcgag atcaaaaaac ctaatcccct ttcgtcatta t 101

<210> 8
<211> 101
<212> DNA
<213> Artificial Sequence

<220>
<223> marker ra25028s01

<400> 8
tttgtttcat atatcttcta ttsatatatg atgtggtaca catatagact kaatgttacc 60

tttgatttta atgagttcaa tctccaaggc aagtagtaat c 101

<210> 9
<211> 101

<212>  DNA
<213>  Artificial Sequence

<220>
<223>  marker ra25042s01

<400>  9
taattccatt tgtctcgtgt gtttctatty ggacacraat gtgtattacc rctaaccata      60

tactcagtac tcaaaatccg ttcatgaatt tcttcacata c                          101


<210>  10
<211>  101
<212>  DNA
<213>  Artificial Sequence

<220>
<223>  marker ra25063s01

<400>  10
tttgagatga agatatgggt tacaagtaag attaatccaa caacggtatc ktggagcaag      60

tttttagaag tggatatgag accattcatt actggttgta a                          101


<210>  11
<211>  45
<212>  DNA
<213>  Artificial Sequence

<220>
<223>  primer for detecting allele X of ra24982s01

<400>  11
gaaggtgacc aagttcatgc tatatgcatc cgagtgtcca ttctc                      45


<210>  12
<211>  45
<212>  DNA
<213>  Artificial Sequence

<220>
<223>  primer for detecting allele Y of ra24982s01

<400>  12
gaaggtcgga gtcaacggat tatatgcatc cgagtgtcca ttctt                      45


<210>  13
<211>  29
<212>  DNA
<213>  Artificial Sequence

<220>
<223>  common primer for detecting the presence or absence of ra24982s01

<400>  13
agaggagtat ttggtcagtg cttatcttt                                        29


<210>  14

<211>  48
<212>  DNA
<213>  Artificial Sequence

<220>
<223>  primer for detecting allele X of ra74607s01

<400>  14
gaaggtgacc aagttcatgc tcgcttagat tttatagaaa aatggccg                    48


<210>  15
<211>  50
<212>  DNA
<213>  Artificial Sequence

<220>
<223>  primer for detecting allele Y of ra74607s01

<400>  15
gaaggtcgga gtcaacggat tatcgcttag attttataga aaaatggcca                  50


<210>  16
<211>  25
<212>  DNA
<213>  Artificial Sequence

<220>
<223>  common primer for detecting the presence or absence of ra74607s01

<400>  16
ctgagcagtc ggtaattagt cgcta                                             25


<210>  17
<211>  51
<212>  DNA
<213>  Artificial Sequence

<220>
<223>  primer for detecting allele X of ra74625s01

<400>  17
gaaggtgacc aagttcatgc taaaagaatt acagtgtaaa tttgtagtat c                51


<210>  18
<211>  53
<212>  DNA
<213>  Artificial Sequence

<220>
<223>  primer for detecting allele Y of ra74625s01

<400>  18
gaaggtcgga gtcaacggat tctaaaagaa ttacagtgta aatttgtagt atg              53


<210>  19
<211>  24
<212>  DNA
<213>  Artificial Sequence

<220>
<223>  common primer for detecting the presence or absence of ra74625s01

<400>  19
ygcgcgggtt atcacctagt gttt                                              24


<210>  20
<211>  45
<212>  DNA
<213>  Artificial Sequence

<220>
<223>  primer for detecting allele X of ra74605s01

<400>  20
gaaggtgacc aagttcatgc tgaatgtttt agagagcttg ctttc                       45


<210>  21
<211>  48
<212>  DNA
<213>  Artificial Sequence

<220>
<223>  primer for detecting allele Y of ra74605s01

<400>  21
gaaggtcgga gtcaacggat tcctgaatgt tttagagagc ttgctttt                    48


<210>  22
<211>  30
<212>  DNA
<213>  Artificial Sequence

<220>
<223>  common primer for detecting the presence or absence of ra74605s01

<400>  22
gactaaaagg agtatggtwa gttaccactt                                        30


<210>  23
<211>  50
<212>  DNA
<213>  Artificial Sequence

<220>
<223>  primer for detecting allele X of ra74601s01

<400>  23
gaaggtgacc aagttcatgc tatcaagaaa aagctatgag tctaaattgc                  50


<210>  24
<211>  51
<212>  DNA
<213>  Artificial Sequence

<220>
<223>  primer for detecting allele Y of ra74601s01

<400>  24
gaaggtcgga gtcaacggat tgatcaagaa aaagctatga gtctaaattg t                51

<210>  25
<211>  26
<212>  DNA
<213>  Artificial Sequence

<220>
<223>  common primer for detecting the presence or absence of ra74601s01

<400>  25
cggttcgtga aagagcttga catgat                                            26

<210>  26
<211>  46
<212>  DNA
<213>  Artificial Sequence

<220>
<223>  primer for detecting allele X of ra74589s02

<400>  26
gaaggtgacc aagttcatgc tcactcaatt tctggccatt gatgac                      46

<210>  27
<211>  46
<212>  DNA
<213>  Artificial Sequence

<220>
<223>  primer for detecting allele Y of ra74589s02

<400>  27
gaaggtcgga gtcaacggat tcactcaatt tctggccatt gatgat                      46

<210>  28
<211>  29
<212>  DNA
<213>  Artificial Sequence

<220>
<223>  common primer for detecting the presence or absence of ra74589s02

<400>  28
cgcgtcttca tatcataaaa ctggcataa                                         29

<210>  29
<211>  49
<212>  DNA
<213>  Artificial Sequence

<220>
<223>  primer for detecting allele X of ra74593s01

<400>  29
gaaggtgacc aagttcatgc tgtagaaaat gaaatcactt atggagagc                   49

<210>  30
<211>  50
<212>  DNA
<213>  Artificial Sequence

<220>
<223>  primer for detecting allele Y of ra74593s01

<400>  30
gaaggtcgga gtcaacggat tggtagaaaa tgaaatcact tatggagagt          50


<210>  31
<211>  29
<212>  DNA
<213>  Artificial Sequence

<220>
<223>  common primer for detecting the presence or absence of ra74593s01

<400>  31
gggattaggt tttttgatct cgatctgtt                                 29


<210>  32
<211>  49
<212>  DNA
<213>  Artificial Sequence

<220>
<223>  primer for detecting allele X of ra25028s01

<400>  32
gaaggtgacc aagttcatgc tatatatgat gtggtacaca tatagactg           49


<210>  33
<211>  49
<212>  DNA
<213>  Artificial Sequence

<220>
<223>  primer for detecting allele Y of ra25028s01

<400>  33
gaaggtcgga gtcaacggat tatatatgat gtggtacaca tatagactt           49


<210>  34
<211>  29
<212>  DNA
<213>  Artificial Sequence

<220>
<223>  common primer for detecting the presence or absence of ra25028s01

<400>  34
ctacttgcct tggagattga actcattaa                                 29


<210>  35

<211>  50
<212>  DNA
<213>  Artificial Sequence

<220>
<223>  primer for detecting allele X of ra25042s01

<400>  35
gaaggtgacc aagttcatgc tggattttga gtactgagta tatggttagt                50


<210>  36
<211>  45
<212>  DNA
<213>  Artificial Sequence

<220>
<223>  primer for detecting allele Y of ra25042s01

<400>  36
gaaggtcgga gtcaacggat tttgagtact gagtatatgg ttagc                     45


<210>  37
<211>  29
<212>  DNA
<213>  Artificial Sequence

<220>
<223>  common primer for detecting the presence or absence of ra25042s01

<400>  37
gctaattcca tttgtctcgt gtgtttcta                                       29


<210>  38
<211>  48
<212>  DNA
<213>  Artificial Sequence

<220>
<223>  primer for detecting allele X of ra25063s01

<400>  38
gaaggtgacc aagttcatgc tagtaagatt aatccaacaa cggtatcg                  48


<210>  39
<211>  50
<212>  DNA
<213>  Artificial Sequence

<220>
<223>  primer for detecting allele Y of ra25063s01

<400>  39
gaaggtcgga gtcaacggat tcaagtaaga ttaatccaac aacggtatct                50


<210>  40
<211>  29
<212>  DNA
<213>  Artificial Sequence

<220>
<223>  common primer for detecting the presence or absence of ra25063s01

<400>  40
accagtaatg aatggtctca tatccactt                                        29

## Description

### FIELD OF THE INVENTION

The invention relates to the field of fungal disease control in *Brassica,* such as *Brassica napus (B. napus).* The invention provides a method of selecting or identifying *Brassica* plants, such as *B. napus* plants, having resistance to Phoma stem canker caused by the fungal pathogen(s) *Leptosphaeria maculans (L. maculans) and*/*or Leptosphaeria biglobosa (L. biglobosa).* Provided are detection tools such as markers and primers for detecting the presence of one or more resistance alleles in *Brassica* plants such as *B. napus* plants or parts thereof, such as tissue or seeds. The invention also relates to methods for providing *Brassica* plants, such as *B. napus* plants having resistance to Phoma stem canker caused by fungal pathogen(s) *L*. *maculans and*/*or L. biglobosa,* as well as uses of the plants and seeds and the methods of the invention.

### BACKGROUND TO THE INVENTION

Phoma stem canker also known as Blackleg, caused by the fungal pathogen(s) *L*. *maculans* and/or *L. biglobosa,* is a major disease of a wide variety of *Brassica* crops including *B. napus* L. (oilseed rape or Canola) and *Brassica oleracea* (cabbage), causing annually major economic losses worldwide, in particular in Europe, Australia, Canada and North America. *L*. *maculans* is especially virulent on *B. napus* which is used worldwide for vegetable oil, biodiesel and meal for stock feed markets.

*B. napus* (2n=38, genome AACC) is an amphidiploid species, which originated from a spontaneous hybridization of *Brassica rapa* L. (syn. *B. campestris;* 2n=20, AA) and *Brassica oleracea* L. (2n=18, CC). *B. napus* contains the complete chromosome sets of these two diploid genomes.

Symptoms of *L. maculans* infection can develop on cotyledons, leaves, pods and stems and include basal stem cankers, small grey lesions on leaves and root rot. The majority of yield loss has been attributed to stem canker.

Leaf lesions typically develop after infection by wind dispersed ascospores and/or water (splash) dispersed conidiospores. Stem symptoms (or cankers) can arise through direct infection of the stems or through systemic growth of the fungus from leaf lesions, through the vascular tissue into the stem. Stem cankers may girdle the stem, which can lead to the lodging of plants (bending of stems near ground level) and plant death. Less severe cankers can cause a restriction in water and nutrient flow, which in turn may lead to shrivelling of seeds and pods. Pod infection can lead to premature podshatter and seed infection.

The production of Phoma stem canker resistant *Brassica* crops such as *B. napus* cultivars is one of the major objectives in crop breeding programs worldwide. Although both chemical management such as spraying of fungicides and cultural management such as crop rotation practices are currently used to reduce yield losses caused by fungal infection, it is considered that the most reliable and likely most environmentally safe method of control to date is genetic resistance. Genetic resistance may be conferred by race-specific resistance (qualitative resistance; R) and/or race non-specific resistance (quantitative resistance; QR) genes.

R gene mediated resistance typically follows the classical gene to gene hypothesis and has major allelic effects which can be tested efficiently and accurately at the cotyledon stage. However a consistent resistance response may not be detected when plants are grown under natural field conditions when constantly challenged with incoming inoculum from diverse races of fungal pathogen such as *L. maculans.* QR is usually controlled by several genes of smaller allelic effect however these QR alleles may together confer more stable resistance at the adult plant stage under field conditions.

Around nineteen resistance loci have been reported from *Brassica* species and of these about eleven genes *(Resistance to L. maculans (Rlm)* genes: *Rlm1, Rlm2, Rlm3, Rlm4, Rlm7, Rlm9, Rlm*11 and *LepR1, LepR2, LepR3* and *LepR4*) are thought to have originated from the A genome of *B. napus* and *Brassica rapa.*

The lack of suitable resistance found in *B. napus* (AACC genome) and the continuous threat of breakdown of resistance when a resistant cultivar is used widespread and over longer time periods necessitates breeders to search for alternative sources of resistance. The main research focus has been on the identification and transfer of resistance alleles from related *Brassica* species, such as *B*. *rapa* (AA), *B*. *oleracea* (CC), *B. nigra* (BB genome), *B. juncea* (AABB genome) and *B*. *carinata* (BBCC).

One genetic approach to provide resistance has been to generate synthetic *B. napus* lines by interspecific hybridization of two diploid species (AA and CC genome) and subsequent *in vitro* culture of embryos and chromosome doubling. Blackleg resistance was introduced into *B. napus* in this way by generating synthetic *B. napus* plants from wild *B. rapa* (AA genome) accessions [Crouch et al., (1994), Plant Breeding 112: 265-278] and wild *B*. *atlantica* (CC genome) accessions [Mithen and Magrath (1992), Plant Breeding 108: 60-68, and Mithen and Herron (1991), Proceedings of the 8th International Rapeseed Congress].

Among a series of synthetic *B. napus* lines with a common C genome but different A genomes two lines were found to be resistant to fungal isolates in glasshouse tests (cotyledon and leaf tests), however the synthetic lines derived from said lines and their F1 hybrids with oilseed rape cultivars, showed high resistance to Blackleg in glasshouse experiments. Only one of these lines also showed resistance in field experiments in England and Australia.

Furthermore, the synthetic B. napus lines described by Crouch and Mithen (supra) were not commercially suitable, as they contained high glucosinolate levels, high erucic acid levels, had poor fertility and suffered from self-incompatibility.

In another example, the open-pollinated B. napus variety Surpass400 was brought onto the market by Pacific Seeds in 2000 in Australia. At that time it received a national Blackleg resistance rating of 9.0 (Australia Blackleg Rating: ABR, Canola Association of Australia), the highest known level of resistance. Surpass400 is a synthetic *B. napus,* derived from interspecific crosses between wild *B*. *rapa* ssp *sylvestris* from Sicily and *B*. *oleracea* ssp *alboglabra* and it was reported that a major dominant allele for Blackleg resistance at the seedling stage was present in Surpass400.

However, by early 2003, reports of a breakdown of Surpass400 resistance surfaced indicating that more virulent strain of the fungus had evolved in just three years.

In a further example of genetic resistance, Yu et al., Theor Appl Gent 2005 Mar; 110(5):969-79 reported resistance loci in *B. napus* populations. A first locus was mapped on linkage group N02 and was designated *LepR1* and a second locus was mapped on linkage group N10 was designated *LepR2. LepR1* and *LepR2* were originally identified in proprietary *B. napus* breeding lines DHP95 ad DHP96 respectively. A third locus, *LepR3* was identified in the *B. napus* cultivar Surpass 400.

With the constant risk of genetic resistance breaking down as a result of changes in the pathogen population, it is desirable for breeders to identify new genetic sources and markers of resistance, methods for transferring these into varieties with high agronomic performance and methods for enhancing durability of resistance.

### SUMMARY OF ASPECTS OF THE INVENTION

The present invention is based, at least in part, on the inventors' determination of the *LepR1* resistance haplotype and markers linked to said haplotype.

Before the present invention, only a few simple sequence repeats (SSR) markers associated with *LepR1* fungal pathogen(s) *L. maculans and*/*or L. biglobosa* resistance were available to breeders. The use of those SSR markers to identify plants with resistance was cost inefficient for breeders and often resulted in plants having poor negative agronomic and/or phenotypic properties caused by linkage drag.

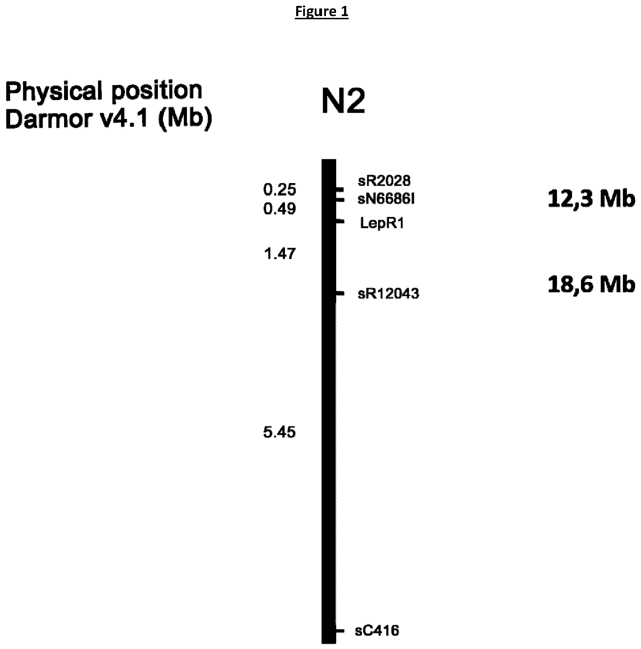

For example, see Figure 1 which shows a genetic map comprising four SSRs on chromosome A02 and *LepR1.* The size of the *LepR1* target region was, based on the public genomic reference Darmor v4.1 at that time about 6Mb.

The use of markers according to the present invention enables the *LepR1* target region associated with resistance to be reduced from about 6Mb to about 90kb.

Thus, the present invention provides markers which allow selection and/or identification of plants having Phoma stem canker resistance and reduced linkage drag. Advantageously, the present invention allows production and/or selection or identification of *B. napus* plants having resistance to fungal pathogen(s) *L. maculans and*/*or L. biglobosa* which maintain commercially acceptable agronomic and/or phenotypic properties. For example, the present invention may allow production and/or selection or identification of plants having reduced or no negative agronomic properties caused by linkage drag.

In one aspect, the present invention provides a method for selecting or identifying a *B. napus* plant having resistance to Phoma stem canker caused by fungal pathogens such as *L*. *maculans* and/or *L. biglobosa,* comprising detecting in said *B. napus* plant, or part thereof (such as tissue or seed), the presence or absence of at least one marker which is on a chromosomal interval between SEQ ID No: 1 and SEQ ID No: 10.

Suitably, said at least one marker may be on a chromosomal interval between SEQ ID No: 2 and SEQ ID No: 9.

Suitably, said at least one marker may be on a chromosomal interval between SEQ ID No: 3 and SEQ ID No: 8.

Suitably, said at least one marker may be on a chromosomal interval between SEQ ID No: 4 and SEQ ID No: 7.

Suitably, said at least one marker may be on a chromosomal interval between SEQ ID No: 5 and SEQ ID No: 6.

Suitably, the method may comprise detecting at least one or more of the following alleles:
a) allele C/T within SEQ ID No: 1 (this allele is also referred to herein as ra24982s01);
b) allele C/T within SEQ ID No: 2 (this allele is also referred to herein as ra74607s01);
c) allele C/G within SEQ ID No: 3 (this allele is also referred to herein as ra74625s01);
d) allele C/T within SEQ ID No: 4 (this allele is also referred to herein as ra74605s01);
e) allele C/T within SEQ ID No: 5 (this allele is also referred to herein as ra74601s01);
f) allele C/T within SEQ ID No: 6 (this allele is also referred to herein as ra74589s02);
g) allele C/T within SEQ ID No: 7 (this allele is also referred to herein as ra74593s01);
h) allele G/T within SEQ ID No: 8 (this allele is also referred to herein as ra25028s01);
i) allele A/G within SEQ ID No: 9 (this allele is also referred to herein as ra25042s01); and/or
j) allele G/T within SEQ ID No: 10 (this allele is also referred to herein as ra25063s01).

Preferably, it is the nucleotide or the respective allele which is associated with the resistance which is detected. In this regard it is to be understood that the method according to the invention encompasses detecting at least one or more of the following alleles:
a) allele y or allele c at ra24982s01 according to SEQ ID No: 1
b) allele y or allele t at ra74607s01 according to SEQ ID No: 2
c) allele s or allele g at ra74625s01 according to SEQ ID No: 3
d) allele y or allele t at ra74605s01 according to SEQ ID No: 4
e) allele y or allele t at ra74601s01 according to SEQ ID No: 5
f) allele y or allele at ra74589s02 according to SEQ ID No: 6
g) allele y or allele c at ra74593s01 according to SEQ ID No: 7
h) allele k or allele t at ra25028s01 according to SEQ ID No: 8
i) allele r or allele g at ra25042s01 according to SEQ ID No: 9
j) allele k or allele g at ra25063s01 according to SEQ ID No: 10.

This method may include detecting
a) the nucleotide or allele c at the position of allele or nucleotide y as given in SEQ ID No: 1
b) the nucleotide or allele t at the position of allele or nucleotide y as given in SEQ ID No: 2
c) the nucleotide or allele g at the position of allele or nucleotide s as given in SEQ ID No: 3
d) the nucleotide or allele t at the position of allele or nucleotide y as given in SEQ ID No: 4
e) the nucleotide or allele t at the position of allele or nucleotide y as given in SEQ ID No: 5
f) the nucleotide or allele c at the position of allele or nucleotide y as given in SEQ ID No: 6
g) the nucleotide or allele c at the position of allele or nucleotide y as given in SEQ ID No: 7
h) the nucleotide or allele t at the position of allele or nucleotide k as given in SEQ ID No: 8
i) the nucleotide or allele g at the position of allele or nucleotide r as given in SEQ ID No: 9
j) the nucleotide or allele g at the position of allele or nucleotide k as given in SEQ ID No: 10.

The detection may involve the marker given above (for example marker ra24982s01 for the detection as described under a) and so on). The use of respective primers is a possibility how the markers may be applied in the context of the method. Respective primers representing the markers are disclosed herein elsewhere. The primers may be tagged, chemically modified or complemented as described herein. The technical parameters according to this paragraph may be used in a method for agricultural cultivation and may be used in a method for combatting *Leptosphaeria maculans* and/or *L. biglobosa.*

Suitably, said method may comprise detecting in said *B. napus* plant, or part thereof (such as tissue or seed), the presence or absence of at least two markers wherein at least one marker is on a first chromosomal interval between SEQ ID No: 1 and SEQ ID No: 5, and at least one of said markers is on a second chromosomal interval between SEQ ID No: 10 and ra74589s02 SEQ ID No: 6.

Suitably,
a) at least one of said markers is on a first chromosomal interval between SEQ ID No: 2 and SEQ ID No: 5, and at least one of said markers is on a second chromosomal interval between SEQ ID No: 9 and SEQ ID No: 6; and/or
b) at least one of said markers is on a first chromosomal interval between SEQ ID No: 3 and SEQ ID No: 5, and at least one of said markers is on a second chromosomal interval between SEQ ID No: 8 and SEQ ID No: 6; and/or
c) at least one of said markers is on a first chromosomal interval between SEQ ID No: 4 and SEQ ID No: 5, and at least one of said markers is on a second chromosomal interval between SEQ ID No: 7 and SEQ ID No: 6; and/or
d) at least one of said markers is on a first chromosomal interval defined by SEQ ID No: 5, and at least one of said markers is on a second chromosomal interval defined by SEQ ID No: 6.

Suitably, said *B. napus* plant may have been obtained by a process of introgressing LepR1 from a donor plant into a recipient *B. napus* plant to produce an introgressed *B. napus* plant. Suitably said donor plant may have resistance to fungal pathogen(s) *L. maculans* and/or *L*. *biglobosa.*

Suitably said introgressed *B. napus* plant may have resistance to fungal pathogen(s) *L*. *maculans* and/or *L. biglobosa.*

Suitably, said donor plant may be *Brassica rapa* subsp. *sylvestris.*

Suitably, said the donor plant may be a wild relative of *B. napus.*

Suitably, said introgressed *Brassica* plant is selected for a recombination event on a chromosomal interval between SEQ ID No: 1 and SEQ ID No: 10. The recombination event may carry the resistance locus or resistance allele according to the invention or may be responsible for the exchange of genetic material in one or both flanking regions of the resistance locus or resistance allele.

Suitably, said introgressed *Brassica* plant is selected for a recombination event on a chromosomal interval between SEQ ID No: 1 and SEQ ID No: 10 and does not retain a second chromosomal interval derived from the donor plant.

In one aspect, said *B. napus* plant or part thereof selected or identified by a method according to the present invention having resistance to fungal pathogen(s) *L. maculans and*/*or L. biglobosa* does not exhibit any negative agronomic properties caused by linkage drag.

In another aspect, the present invention provides a kit of primers for identifying a *B. napus* plant having resistance to Phoma stem canker (such as resistance to fungal pathogen(s) *L. maculans* and/or *L. biglobosa*) comprising:
a) a set of primers capable of identifying the presence or absence of marker ra24982s01, comprising a primer comprising SEQ ID No: 11 or a primer comprising SEQ ID No: 12 and a primer comprising SEQ ID No. 13 and/or
b) a set of primers capable of identifying the presence or absence of marker ra74607s01, comprising a primer comprising SEQ ID No: 14 or a primer comprising SEQ ID No: 15 and a primer comprising SEQ ID No. 16; and/or
c) a set of primers capable of identifying the presence or absence of marker ra74625s01, comprising a primer comprising SEQ ID No: 17 or a primer comprising SEQ ID No: 18 and a primer comprising SEQ ID No. 19; and/or
d) a set of primers capable of identifying the presence or absence of marker ra74605s01, comprising a primer comprising SEQ ID No: 20 or a primer comprising SEQ ID No: 21 and a primer comprising SEQ ID No. 22; and/or
e) a set of primers capable of identifying the presence or absence of marker ra74601s01, comprising a primer comprising SEQ ID No: 23 or a primer comprising SEQ ID No: 24 and a primer comprising SEQ ID No. 25; and/or
f) a set of primers capable of identifying the presence or absence of marker ra74589s02, comprising a primer comprising SEQ ID No: 26 or a primer comprising SEQ ID No: 27 and a primer comprising SEQ ID No. 28; and/or
g) a set of primers capable of identifying the presence or absence of marker ra74593s01, comprising a primer comprising SEQ ID No: 29 or a primer comprising SEQ ID No: 30 and a primer comprising SEQ ID No. 31; and/or
h) a set of primers capable of identifying the presence or absence of marker ra25028s01, comprising a primer comprising SEQ ID No: 32 or a primer comprising SEQ ID No: 33 and a primer comprising SEQ ID No. 34; and/or
i) a set of primers capable of identifying the presence or absence of marker ra25042s01, comprising a primer comprising SEQ ID No: 35 or a primer comprising SEQ ID No: 36 and a primer comprising SEQ ID No. 37 and/or
j) a set of primers capable of identifying the presence or absence of marker ra25063s01, comprising a primer comprising SEQ ID No: 38 or a primer comprising SEQ ID No: 39 and a primer comprising SEQ ID No. 40.

In a further aspect, the present invention provides the use of a plant selected by a method according to the present invention for producing an oilseed rape oil or an oilseed rape seed cake.

In another aspect, the present invention provides the use of at least one marker as described herein (for example as defined in any of claims 1 to 13), or a marker selected from SEQ ID No: 1-10, or a kit of primers according to the present invention, to select a *B. napus* plant having resistance to Phoma stem canker (such as resistance to fungal pathogen(s) *L. maculans and*/*or L. biglobosa).* Suitably, the *B. napus* plant or part thereof selected or identified by a method according to the present invention having resistance to Phoma stem canker (such as fungal pathogen(s) *L. maculans and*/*or L. biglobosa)* may not exhibit any negative agronomic and/or phenotypic properties caused by linkage drag. In this respect the invention encompasses such plants which carry the resistance locus or resistance allele according to the invention and provide the same yield (for example the same number of kernels or the same oil mass) as plants which do not carry the resistance locus or resistance allele. Furthermore, the invention also encompasses methods for the identification and / or selection of such plant as described herein.

The methods for selection, detection or identification described herein may start with a step of provision of a plant, plant part, plant tissue or seed and may continue with a subsequent step of isolating DNA from that plant, plant part, plant tissue or seed. The subsequent step of detection, or identification my be carried out on the isolated DNA. For this detection or identification markers as discloses herein may be applied. Preferably, at least one, at least two, at least three, at least four, at least five or at least six markers as disclosed herein are applied. The step of detection or identification may include the creation of electronically transmittable and / or electronically storable data representing the detection or identification of the presence of the respective nucleic acid molecule or the nucleotide sequence or the presence of DNA or a genomic section which implies the resistance according to the invention to the plant comprising the detected or identified genetic material. The data may be stored on a computer readable medium. The method may include a step of selecting the resistant plant in which the respective genetic material has been identified or detected. In a preferred embodiment only those plants are selected with are homozygous for the respective resistance-conferring genetic material. The resistance-conferring genetic material may be an allele detectable may the markers and methods disclosed herein.

### DESCRIPTION OF THE FIGURES

**Figure 1** - shows a genetic map of SSRs projected on physical map of Darmorv4.1.
**Figure 2** - shows fine mapping of the A02 chromosome target region for the LepR1 resistance locus. B= non-LepR1 allele, G= heterozygous allele status. The frame indicates the target region here the gene is located, based on the recombinants shown in the figure compared to resistance and susceptibility (RES/SUS). The markers shown in the frame may be used for selection of plants having fungal resistance. The RA numbers refer to internal processing numbers.
**Figure 3** **-** shows scoring of (A) *L. maculans* and (B) *L. biglobosa* on a set of lines carrying different types of resistance.

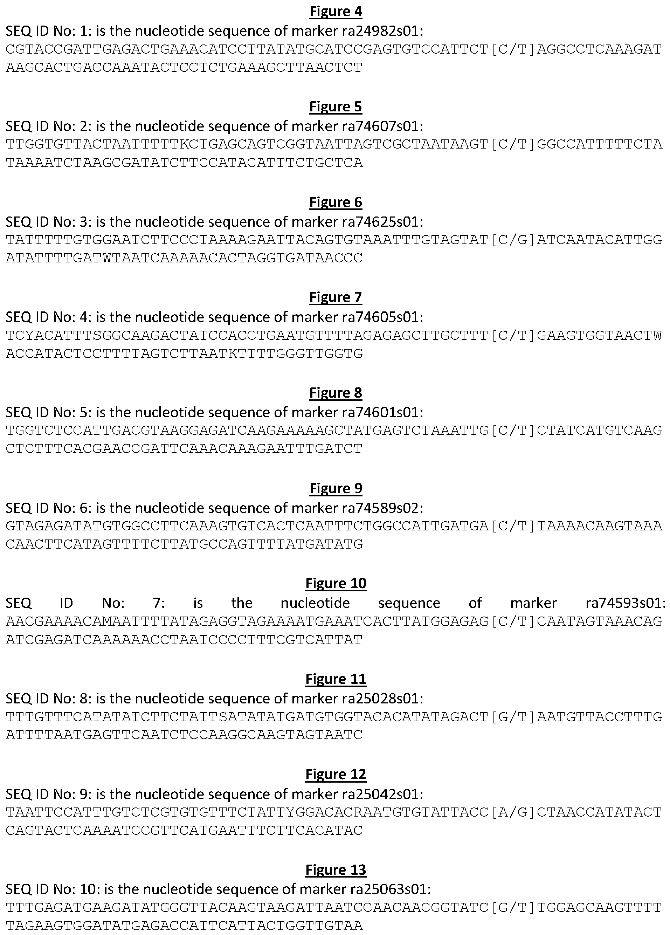

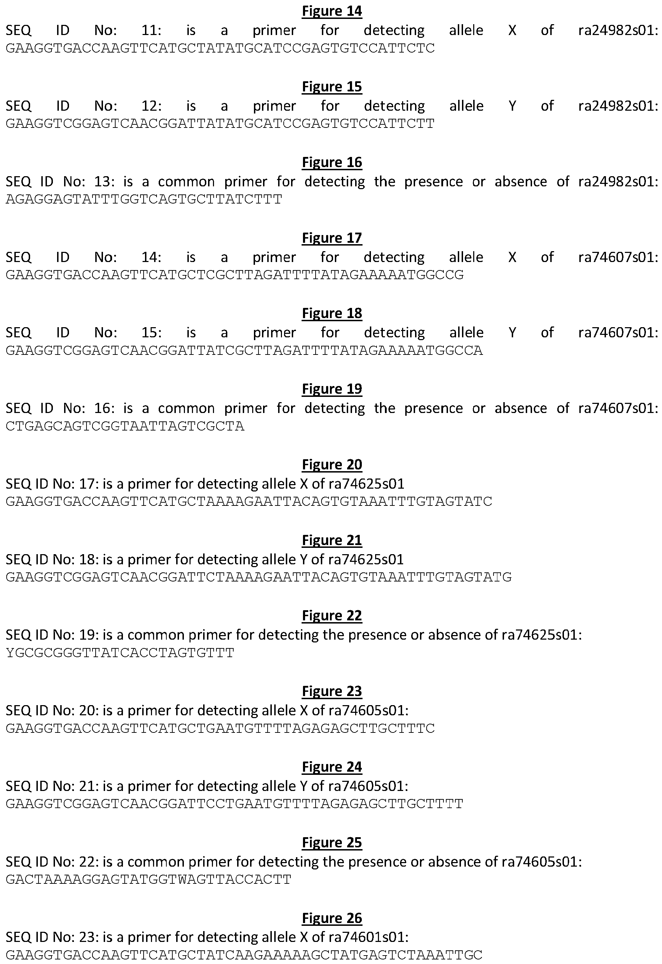

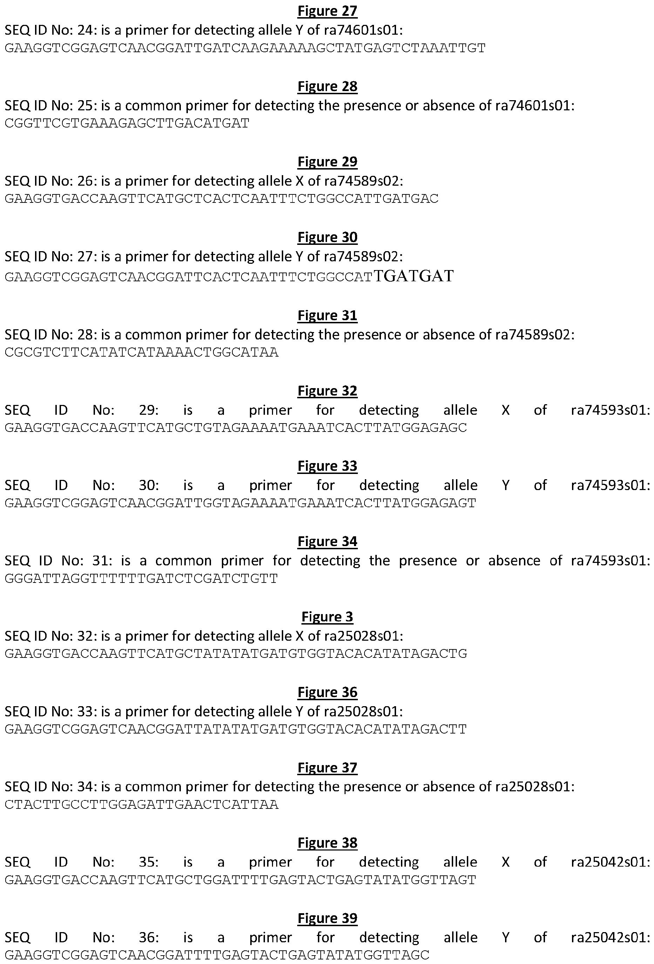

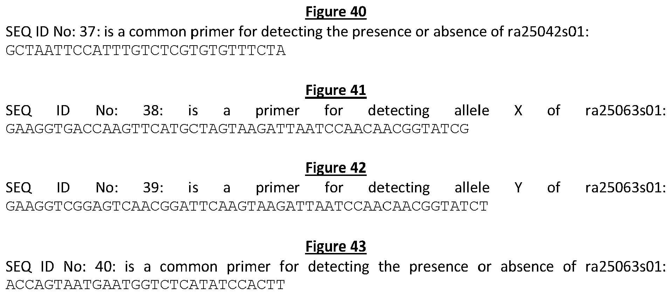

**Figures 4 to 43** **-** show the sequences of SEQ ID Nos 1 to 40 as described below.

### DESCRIPTION OF THE SEQUENCES

SEQ ID No: 1: is a nucleotide sequence comprising marker ra24982s01.
SEQ ID No: 2: is a nucleotide sequence comprising marker ra74607s01.
SEQ ID No: 3: is a nucleotide sequence comprising marker ra74625s01.
SEQ ID No: 4: is a nucleotide sequence comprising marker ra74605s01.
SEQ ID No: 5: is a nucleotide sequence comprising marker ra74601s01.
SEQ ID No: 6: is a nucleotide sequence comprising marker ra74589s02.
SEQ ID No: 7: is a nucleotide sequence comprising marker ra74593s01.
SEQ ID No: 8: is a nucleotide sequence comprising marker ra25028s01.
SEQ ID No: 9: is a nucleotide sequence comprising marker ra25042s01.
SEQ ID No: 10: is a nucleotide sequence comprising marker ra25063s01.
SEQ ID No: 11: is a primer for detecting the resistance allele of ra24982s01.
SEQ ID No: 12: is a primer for detecting the sensitive allele of ra24982s01.
SEQ ID No: 13: is a common primer for detecting the presence or absence of ra24982s01.
SEQ ID No: 14: is a primer for detecting the sensitive allele of ra74607s01.
SEQ ID No: 15: is a primer for detecting the resistance allele of ra74607s01.
SEQ ID No: 16: is a common primer for detecting the presence or absence of ra74607s01. SEQ ID No: 17: is a primer for detecting the sensitive allele of ra74625s01.
SEQ ID No: 18: is a primer for detecting the resistance allele of ra74625s01.
SEQ ID No: 19: is a common primer for detecting the presence or absence of ra74625s01.
SEQ ID No: 20: is a primer for detecting the sensitive allele of ra74605s01.
SEQ ID No: 21: is a primer for detecting the resistance allele of ra74605s01.
SEQ ID No: 22: is a common primer for detecting the presence or absence of ra74605s01.
SEQ ID No: 23: is a primer for detecting the sensitive allele of ra74601s01.
SEQ ID No: 24: is a primer for detecting the resistance allele of ra74601s01.
SEQ ID No: 25: is a common primer for detecting the presence or absence of ra74601s01.
SEQ ID No: 26: is a primer for detecting the resistance allele of ra74589s02.
SEQ ID No: 27: is a primer for detecting the sensitive allele of ra74589s02.
SEQ ID No: 28: is a common primer for detecting the presence or absence of ra74589s02.
SEQ ID No: 29: is a primer for detecting the resistance allele of ra74593s01.
SEQ ID No: 30: is a primer for detecting the sensitive allele of ra74593s01.
SEQ ID No: 31: is a common primer for detecting the presence or absence of ra74593s01.
SEQ ID No: 32: is a primer for detecting the sensitive allele of ra25028s01.
SEQ ID No: 33: is a primer for detecting the resistance allele of ra25028s01.
SEQ ID No: 34: is a common primer for detecting the presence or absence of ra25028s01.
SEQ ID No: 35: is a primer for detecting the sensitive allele of ra25042s01.
SEQ ID No: 36: is a primer for detecting the resistance allele of ra25042s01.
SEQ ID No: 37: is a common primer for detecting the presence or absence of ra25042s01.
SEQ ID No: 38: is a primer for detecting the resistance allele of ra25063s01.
SEQ ID No: 39: is a primer for detecting the sensitive allele of ra25063s01.
SEQ ID No: 40: is a common primer for detecting the presence or absence of ra25063s01.

### DETAILED DESCRIPTION

### Resistance

In one aspect of the present invention, there is provided a method for selecting or identifying a *B. napus* plant having resistance to Phoma stem canker.

In other words, the *B. napus* plant has resistance to the fungal pathogens which cause Phoma stem canker. The *B. napus* plant is resistant to Phoma stem canker disease.

"Phoma stem canker" also referred to as "Blackleg" is an economically important disease of crucifers, including *B*. *napus, B. juncea, B. oleracea* and *B*. *rapa.* Two fungal pathogens form a species complex which causes Phoma stem canker: *L*. *maculans,* which is associated with damaging stem base cankers; and *L. biglobosa,* which is often associated with less damaging upper stem lesions.

Isolates of *L. biglobosa* are often found in association with *L. maculans,* but they can be found independently of one another. Blackleg isolates may be characterized based on pathogenicity tests and molecular phylogenetic analysis (see for example, Z. Zou et al., Int. J. Mol. Sci. 2019, 20, 1668) which is incorporated herein by reference. *L*. *maculans* isolates can be classified into different pathogenicity groups (PG), depending on their specific interactions with *B. napus* cultivars Westar, Galcier and Quinta [Mengistu et al., (1991), Plant Disease 75:1279-1282]. PG4 isolates cause sporulating lesions on all three cultivars, while PG3 isolates cause a resistance reaction on cotyledons of Quinta, and PG2 isolates cause a resistance reaction on cotyledons of Quinta and Glacier. PG1 isolates are non-pathogenic on these hosts. PG2, PG3 and PG4 isolates are referred to as 'highly aggressive' or 'highly virulent' or 'strongly pathogenic' isolates, and PG1 isolates are typically referred to as 'non-aggressive' or 'non-virulent' or 'weakly pathogenic'. The highly aggressive group has also been distinguished from the weakly aggressive group by its production of toxins (Tox⁺ isolates vs Tox° isolates). Tox° isolates have been found to cause necrosis of the pith, unaccompanied by external symptoms, and are further distinguished into three groups, NA1, NA2 and NA3 and it has been suggested that NA1 isolates are predominant in Europe and NA2 isolates are more important in Canada.

In one aspect, the present invention provides a method for selecting or identifying a *B. napus* plant having resistance *to L. maculans.* In other words, the plant is resistant to infection by *L. maculans.* In one aspect, the present invention provides a method for selecting or identifying a *B. napus* plant having resistance to *L. biglobosa.* In other words, the plant is resistant to infection by *L. biblobosa.*

"Pathogen resistance", "fungal resistance" and "disease resistance" mean that the plant avoids or exhibits reduced disease symptoms that are the outcome of plant-pathogen interactions. In other words, pathogens are prevented from causing plant diseases and the associated disease symptoms, or the disease symptoms caused by the pathogen are reduced, such as, for example the reduction of stress and associated loss.

As used herein, "fungal resistance" refers to enhanced resistance or tolerance to a fungal pathogen when compared to a plant which does not contain at least one of the markers described herein. Resistance may vary from an increase in tolerance to the effects of the fungal pathogen (e.g., partial inhibition of the pathogen) to total resistance such that the plant is unaffected by the presence of the fungal pathogen.

"Resistance" of plants comprising a certain resistance gene, or markers as described herein refers to a reduction in damage caused by fungal infection compared to damage caused to control plants. Damage can be assessed as, for example, the number and size of leaf symptoms, frequency and severity of stem symptoms, lodging of plants due to stem infection.

In particular, the reduction in damage is manifested in a reduced yield loss when plants are grown under disease pressure in the field, compared to control plants. Such reduction in yield loss can, for example, be due to the fact that the infection, reproduction, spread or survival of the fungus is reduced or prevented in plants with increased resistance. Increased resistance may also refer to plants that are completely resistant, i.e., plants on which no disease symptoms are found or plants which get the highest resistance scores in available Blackleg scoring or rating assays, e.g., Khangura et al. (2003, Department of Agriculture, Western Australia, Farmnote No. 6/2003, ISSN 0726-934X) which is incorporated herein by reference.

Enhanced resistance can also be assessed in bioassays carried out in controlled environments, such as growth chambers, but ideally are confirmed in field trials, as controlled environment assessments may not reflect field conditions. This may be due to the fact that few, single spore isolates of the fungus are normally tested in bioassays, while in the field much larger variation in the pathogen population exists.

In one aspect, resistance is measured under field conditions. Suitably, methods according to the present invention may select or identify a *B. napus* plant having increased resistance to Phoma stem canker when grown in the field, or under field conditions.

For example, resistance may be measured using the PG2 scoring system or the Premature Ripening (PMR) scoring system described in Example 2.

For example, using the PG2 scoring system described in Example 2, a *B. napus* plant having at least one marker according to the present invention may have a reduction in PG2 score of at least 10%, at least 20%at least 30%, at least 40%, at least 50%, at least 60%, at least 70% or at least 80% when compared to control plants or equivalent plants that are susceptible to infection (for example which do not have at least one marker according to the present invention and lack the *LepR1* resistance locus or gene).

A *B. napus* plant identified by the present invention may have a resistance score of about 6.0 - 9.0 when measured with the Australian Blackleg Rating (ABR) (Marcroft et al., Aust. J. Exp. Agric. 42: 587-594, incorporated herein by reference). For example, when assessing resistance on a scale of 1.0 to 9.0, whereby 1.0 is the most susceptible and 9.0 is the most resistant phenotype, a plant which does not have at least one maker according to the present invention may have a score of less than 5, for example from about 1.0 - 2.0 wherein a plant according to the present invention may have a score of at least five, for example 5.0 to 10. or at 5.0 to 8.0.

In one aspect, a *B. napus* plant identified by a method according to the present invention having resistance to Phoma stem canker has agronomically acceptable yield. In one embodiment the yield is measured as kernel mass (dt/ha).

When a method according to the present invention is used to select or identify plants from a breeding program, the method allows the identification of progeny which have resistance to Phoma stem canker and which have agronomically acceptable yield. For example, the method may be used to select progeny which will have a yield comparable to the recipient plant, for example at least 80%, at least 85%, at least 90%, at least 95% of the yield of a recipient plant (when grown in similar field conditions).

In one aspect, an introgressed (or backcrossed) *B. napus* plant identified by a method according to the present invention having resistance to Phoma stem canker has at least a agronomically acceptable yield which means that it can be used profitably in agriculture.

It is understood that environmental conditions, such as location, weather conditions and disease pressure, as well as individual perception of the person assessing disease symptoms, can have an effect on the scoring of Blackleg resistance. Hence, the skilled person understands that variation in these factors in comparative tests should be minimized.

Any other resistance ratings known in the art can be applied in accordance with this invention to compare the plants of the invention with control plants.

Enhanced or improved fungal resistance may refer to an increased level of resistance against a particular fungal pathogen (such as *L. maculans and*/*or L. biglobosa)* or against a wider spectrum of fungal pathogens.

In particular the present invention refers to *B. napus* plants which are resistant to infection by fungal pathogens (such as by *L. maculans and*/*or L.a biglobosa)* or which have enhanced resistance to infection by *L. maculans and*/*or L. biglobosa* as a result of the *LepR1* locus.

Accordingly, said plants typically exhibit increased resistance when compared to equivalent plants that are susceptible to infection by *L. maculans and*/*or L. biglobosa* because they lack the *LepR1 resistance* locus.

In one aspect, the present invention provides a method for selecting or identifying a *B. napus* plant having resistance which is associated with or caused by the *LepR1* locus.

A "locus" as used herein is the position that a gene occupies on a chromosome.

A *"LepR1* locus" refers to the position on the A02 linkage group/chromosome where a Phoma stem canker "resistance gene" is located. Included in this definition is the fragment (or segment) of genomic DNA of the chromosome on which the *LepR1* resistance locus is located.

The *LepR1* locus referred to herein is located within a chromosomal interval which corresponds to the interval between SEQ ID No: 1 and SEQ ID No: 10, such as between SEQ ID No: 2 and SEQ ID No: 9, such as between SEQ ID No: 3 and SEQ ID No: 8, such as between SEQ ID No: 4 and SEQ ID No: 7, such as between SEQ ID No: 5 and SEQ ID No: 6. Suitably, the *LepR1* locus referred to herein is located within a chromosomal interval which corresponds to the interval between ra24982s01 and ra25063s01, such as between ra74607s01 and ra25042s01, such as between ra74625s01 and ra25028s01 , such as between ra74605s01 and ra74593s01, such as between ra74601s01 and ra74589s02.

In other words, the *LepR1* resistance locus referred to herein is located within a chromosomal interval which corresponds to positions 17985071 to 18087922 of the Darmor v4.1 reference genome.

Version 4.1 of the *B*. *napus* "Darmor-*bzh*" reference genome sequence assembly was published in 2014; see for example Chalhoub et al., 2014 Science 345:950-953 which is incorporated herein by reference.

Other published *B. napus* genome assemblies include Darmor-bzh v8.1 and Tapidor (both described by Bayer et al., (2017) 1027 Plant Biotechnol. J. 15 1602-1610); ZS11 (Sun et al., (2017) Plant J. 92 452-468); and the winter cultivar Express 617 (Lee et al., (2020) Front Plant Sci. 11: 496) all of which are incorporated herein by reference.

One skilled in the art is able to determine whether a chromosomal interval corresponds to the recited intervals using methods available in the art for example by sequence alignment and comparison tools.

A "resistance gene" as used herein refers to a DNA sequence which confers, or is associated with, enhanced resistance of a plant, preferably a *B. napus* plant, to Phoma stem canker, compared to a plant lacking the resistance gene(s) or having a non-functional (or inactivated) form of the gene(s).

*"LepR1"* is a "Phoma stem canker resistance locus" which is linked with markers on a chromosomal interval between SEQ ID No: 1 and SEQ ID No: 10 for example linked with markers ra24982s01, ra74607s01 , ra74625s01 , ra74605s01, ra74601s01, ra74589s02, ra74593s01, ra25028s01, ra25042s01 and ra25063s01.

In one aspect, the *LepR1* resistance locus or *LepR1* resistance gene can be transferred from a donor plant to a recipient plant such as different varieties of *B. napus,* and even to different species of *Brassica* plants, e.g. *B. juncea,* e.g., using the molecular markers of this invention.

Suitably, transfer of the *LepR1* resistance locus or gene may increase the resistance of the recipient plant to Phoma stem canker.

A *B. napus* plant having at least one marker according to the present invention may have increased resistance to Phoma stem canker compared to a comparable plant such as a *B*. *napus* plant which does not have at least one marker according to the present invention.

Without wishing to be bound by theory, the markers described herein are linked with the *LepR1* resistance locus or *LepR1* resistance gene; thus a *B. napus* plant having at least one marker according to the present invention comprises the *LepR1* resistance locus or *LepR1* resistance gene and has increased resistance to Phoma stem canker when compared to a comparable plant, such as a *B. napus* plant which does not have at least one marker according to the present invention and which does not comprise the *LepR1* resistance locus or *LepR1* resistance gene.

### Marker

In one aspect, the present invention provides markers for selecting or identifying a *B. napus* plant having resistance to Phoma stem canker.

In another aspect, the present invention provides a method for selecting or identifying a *B*. *napus* plant having resistance to Phoma stem canker, comprising detecting in said *B. napus* plant, or part thereof (such as tissue or seed), the presence or absence of at least one marker described herein.

A "marker" as used herein refers to a molecular marker which is a measurable, genetic characteristic with a fixed position in the genome, which is normally inherited in a Mendelian fashion, and which can be used for mapping of a trait of interest.

The nature of the marker is dependent on the molecular analysis used and can be detected at the DNA, RNA or protein level.

Numerous types of markers are available including but not limited to Single Nucleotide Polymorphisms (SNPs); microsatellites which are also termed SSR's; expressed sequence tags (ESTs) and SSRs derived from EST sequences; RFLP restriction fragment length polymorphisms (RFLP); random amplified polymorphic DNA (RAPD) and amplified fragment length polymorphism (AFLP). Oligonucleotides or primers may be used as markers as long as they can be used to produce a detectable signal (like for example an amplification product). Furthermore, the markers, primers and oligonucleotides according to the invention may be connected to a fluorescent dye in order to generate a fluorescence signal, e.g., under excitation via light of the corresponding wavelength. The fluorescent dye may be a fluorochrome. The markers, primers or oligonucleotides according to the invention may be coupled with other compounds that are suitable for generating a signal. Such markers, primers or oligonucleotides do not occur in nature and also cannot be isolated from nature. The following is executed to produce such marked oligonucleotides: DNA may be marked bio-orthogonally. For this, DNA may be marked in vivo or in vitro with nucleoside analogs, which, for example, may subsequently be coupled with a fluorophore per Staudinger reaction. In addition to this, DNA may also be chemically provided with fluorophores. Markers, primers and oligonucleotides may be marked via a phosphoramidite synthesis with fluorophores that, for example, are used in QPCR, DNA sequencing, and in situ hybridization. Furthermore, DNA may be generated enzymatically in the course of a polymerase chain reaction (PCR) with fluorescent nucleotides, or be marked with a ligase or a terminal deoxynucleotidyl transferase. DNA may also be detected indirectly via a biotinylation and fluorescent avidin. For couplings, fluorescein, fluorescent lanthanides, gold nanoparticles, carbon nanotubes, or quantum dots, among other things, are used as fluorophores. One of the most commonly used fluorescent substances is FAM (carboxyfluorescein). Consequently, oligonucleotides and, in particular, primers that possess a FAM marking are encompassed by the invention. FAM is preferably present as 6-FAM, wherein - depending upon the desired wavelength of the emission and excitation - other FAM variants, e.g., 5-FAM, may, however, also be used. Examples of additional fluorescence markers are AlexaFluor, ATTO, Dabcyl, HEX, Rox, TET, Texas Red, and Yakima Yellow. Depending upon the field of use, the primers or oligonucleotides may be furnished with modifications of the bases or of the sugar phosphate spine. Among these are, among others, amino-dT, azide-dT, 2-aminopurine,5-Br-dC, 2'-deoxyinosine (INO), 3'-deoxy-A, C, G, 5-Met-dC, 5-OH-Met-dCN6-Met-dA, and others. If mapping methods are to be applied appropriate primers, oligonucleotides are dictated by the mapping method to be used.

In one aspect, at least one marker according to the present invention is a SNP marker.
SNP markers detect single base pair nucleotide substitutions. SNPs are the most abundant of the molecular markers, thus having the potential to provide the highest genetic map resolution. SNPs can be assayed at an even higher level of throughput than SSRs, require low amounts of DNA and are relatively low cost systems. Several methods are available for SNP genotyping, including but not limited to, hybridization, primer extension, oligonucleotide ligation, nuclease cleavage, minisequencing and coded spheres.

The process or method explained above concerning the polymerase chain reaction (PCR) may involve two allele-specific forward primers (or their use) and wherein the detection step involves fluorescence resonant energy transfer (FRET) wherein the presence, absence or kind of the fluorescence is determined by a sensor. The sensor signal may be turned into electronically transmittable and / or electronically storable data representing the detection of the presence of the nucleic acid molecule or the nucleotide sequence. In addition, the electronically transmittable and / or electronically storable data may be stored on a computer readable medium. The kind of the fluorescence may be its colour / wave length or the specific dye responsible for the fluorescence (like FAM or HEX). The determination of the fluorescence by the sensor may be an end-point fluorescent read.

What is to be understood by "hybridizing" or "hybridization" is a process in which a single-stranded nucleic acid molecule binds to a nucleic acid strand that is complementary to the greatest possible extent, i.e., forms base pairs with this. Standard methods for hybridization are described in, for example, Sambrook et al., Molecular Cloning: A Laboratory Manual, 3rd ed., Cold Spring Harbor Laboratory Press, Cold Spring Harbor, NY, 2001. What is preferably understood by this is that at least 60% - more preferably, at least 65%, 70%, 75%, 80%, or 85%, and, particularly preferably, 90%, 91%, 92%, 93%, 94%, 95%, 96%, 97%, 98%, or 99% - of the bases of the nucleic acid molecule form a base pairing with the nucleic acid strand that is complementary to the greatest possible extent. The possibility of such an annealing depends upon the stringency of the hybridization conditions. The term, "stringency," relates to the hybridization conditions. High stringency is present when a base pairing is made more difficult; low stringency is present if a base pairing is made easier. For example, the stringency of the hybridization conditions depends upon the salt concentration or ionic strength and the temperature. In general, the stringency may be increased by increasing the temperature and/or decreasing the salt content. What are to be understood by "stringent hybridization conditions" are those conditions given which a hybridization predominantly occurs only between homologous nucleic acid molecules. The term, "hybridization conditions," thereby relates not only to the conditions prevailing in the actual addition of the nucleic acids, but also to the conditions prevailing in the following washing steps. For example, stringent hybridization conditions are conditions under which, predominantly, only those nucleic acid molecules hybridize that have at least 70% - preferably, at least 75%, at least 80%, at least 85%, at least 90%, or at least 95% - sequence identity. Stringent hybridization conditions are, for example: hybridization in 4 x SSC at 65 °C, and subsequent repeated washing in 0.1 x SSC at 65 °C for approximately 1 hour in total. A hybridization preferably occurs under stringent conditions. Sequences which hybridize under stringent conditions to the resistance locus are part of the invention.

SNPs can be used to describe a haplotype for any particular genotype. The SNP marker may be for example a KASPAR marker as described herein elsewhere.

In one aspect, at least one marker according to the present invention is an SSR marker.

SSRs are relatively short runs of tandemly repeated DNA with lengths of 6 bp or less. Polymorphisms arise due to variation in the number of repeat units. The variation in repeat length may be detected by designing PCR primers to the conserved non-repetitive flanking regions. SSRs are highly suited to mapping and MAS as they are multi-allelic, codominant, reproducible and amenable to high throughput automation. Scoring or marker genotype is based on the relative size of the amplified fragment.

In one aspect, at least one marker according to the present invention is a FLP marker.

Various types of FLP markers can be generated. Typically, amplification primers are used to generate fragment length polymorphisms. Such FLP markers are similar to SSR markers, except that the region amplified by the primers is not usually a highly repetitive region. However, the amplified region, or amplicon, will have sufficient variability among germplasm, often due to insertions or deletions, such that the fragments generated by the amplification primers can be distinguished among polymorphic individuals.

For markers to be useful, they need to detect differences, or polymorphisms, within the population being monitored. For molecular markers, this means differences at the DNA level due to polynucleotide sequence differences (e.g. SSRs, RFLPs, SNPs). The genomic variability can be of any origin, for example, insertions, deletions, duplications, repetitive elements, point mutations, recombination events, or the presence and sequence of transposable elements. Molecular markers can be derived from genomic or expressed nucleic acids (e.g., ESTs). ESTs are generally well conserved within a species, while other regions of DNA (typically non-coding) tend to accumulate polymorphism, and therefore, can be more variable between individuals of the same species. A number of *Brassica* molecular markers are known in the art, and are published or available from various sources, such as the *Brassica* Information Portal of the Earlham Institute.

In another aspect, the present invention provides molecular marker probes for use in methods according to the present invention.

A "molecular marker probe" is a nucleic acid sequence or molecule that can be used to identify the presence of a marker or marker locus e.g. a nucleic acid probe that is complementary to a marker locus sequence.

In some aspects, a marker probe refers to a probe of any type that is able to distinguish *(i.e.,* genotype) the particular allele that is present at a marker locus. Nucleic acids are "complementary" when they specifically hybridize in solution, e.g., according to Watson-Crick base pairing rules.

A marker is said to be "linked" to or "associated with" a gene, locus or trait, if the marker and the gene, locus or trait have a greater association in inheritance than would be expected from independent assortment, for example, the marker and the locus may co-segregate in a segregating population and are located on the same chromosome.

"Linkage" refers to the genetic distance of the marker to the locus (or two loci or two markers to each other). The closer the linkage, the smaller the likelihood of a recombination event taking place which separates the marker from the gene or locus. A genetic distance (map distance) is calculated from recombination frequencies and is expressed in centiMorgans (cM).

Loci or genes are typically considered genetically linked if the recombination frequency between them is less than about 50% as determined on a single meiosis map. They are progressively more linked if the recombination frequency is about 40%, about 30%, about 20%, about 10% or less, as determined on a single meiosis map. Two or more genes are physically linked (or syntenic) if they have been demonstrated to be on a single piece of DNA; such as a chromosome. Genetically linked genes will be physically linked (or syntenic), but the exact physical distance (number of nucleotides) may not have been demonstrated yet.

As used herein, the term "closely linked" refers to genetically linked markers within 15 cM or less, including without limitation 12 cM or less, 10 cM or less, 8 cM or less, 7 cM or less, 6 cM or less, 5 cM or less, 4 cM or less, 3 cM or less, 2 cM or less, 1 cM or less and .5 cM or less, as determined on the genetic map.

As used herein "chromosomal interval" refers to a contiguous linear span of genomic DNA that resides *in planta* on a single chromosome, usually defined with reference to two markers. Preferably, the interval does include the two flanking markers.

In one aspect, a chromosomal interval refers to a sequence comprising any two of SEQ ID Nos: 1 to 10 and the sequence in between said sequences.

For example, the chromosomal interval may wholly encompass SEQ ID No: 1 and SEQ ID No: 10 and all of the sequence between SEQ ID No:1 and SEQ ID NO: 10. Suitably, the chromosomal interval may wholly encompass SEQ ID No: 5 and SEQ ID No: 6 and all of the sequence between SEQ ID No: 5 and SEQ ID No: 6.

In one aspect, the chromosomal interval refers to the alleles within any two of SEQ ID Nos 1 to 10 and the sequence in between said alleles.

For example, the chromosomal interval may encompass allele ra24982s01 and allele ra25063s01 and the sequence between said alleles. Suitably, the chromosomal interval may wholly encompass allele ra74601s01 and allele ra74589s02 and all of the sequence between said alleles.

As used herein, a "recombination event" refers to the occurrence of recombination between homologous chromosomes, and refers to a specific chromosomal location where such a recombination has occurred (e.g. a recombination of a chromosomal interval internal to the end points of the chromosome will have a recombination event at each end of the chromosomal interval).

In the context of the present invention, the chromosomal interval is between SEQ ID No: 1 and SEQ ID No: 10. The interval may encompass SEQ ID No: 1 and SEQ ID No: 10 and any sequence in between. The interval may encompass the allele identified within SEQ ID No: 1 (ra24982s01) and the allele identified within SEQ ID No: 10 (ra25063s01) and any sequence in between.

Suitably, the chromosomal interval may be between SEQ ID No: 2 and SEQ ID No: 9. The interval may encompass SEQ ID No: 2 and SEQ ID No: 9 and any sequence in between. The interval may encompass the allele identified within SEQ ID No: 2 (ra74607s01) and the allele identified within SEQ ID No: 9 (ra25042s01) and any sequence in between.

Suitably, the chromosomal interval may be between SEQ ID No: 3 and SEQ ID No: 8. The interval may encompass SEQ ID No: 3 and SEQ ID No: 8 and any sequence in between. The interval may encompass the allele identified within SEQ ID No: 3 (ra74625s01) and the allele identified within SEQ ID No: 8 (ra25028s01) and any sequence in between.

Suitably, the chromosomal interval may be between SEQ ID No: 4 and SEQ ID No: 7. The interval may encompass SEQ ID No: 4 and SEQ ID No: 7 and any sequence in between. The interval may encompass the allele identified within SEQ ID No: 4 (ra74605s01) and the allele identified within SEQ ID No: 7 (ra74593s01) and any sequence in between.

Suitably, the chromosomal interval may be between SEQ ID No: 5 and SEQ ID No: 6. The interval may encompass SEQ ID No: 5 and SEQ ID No: 6 and any sequence in between. The interval may encompass the allele identified within SEQ ID No: 5 (ra74601s01) and the allele identified within SEQ ID No: 6 (ra74589s02) and any sequence in between.

Suitably, the chromosomal interval may be defined by SEQ ID No: 5.

Suitably, the chromosomal interval may be defined by SEQ ID No: 6.

Suitably, at least one marker may be closely linked to any one of SEQ ID No:s1 to 10. Suitably, at least one marker may be closely linked to ra74601s01, the allele identified within SEQ ID No: 5 and/or ra74589s02, the allele identified within SEQ ID No: 6.

In one aspect, at least one marker may be on a chromosomal interval between SEQ ID No: 1 and SEQ ID No: 10; and be closely linked to

SEQ ID No: 5, or ra74601s01, the allele identified within SEQ ID No: 5 and/or closely linked to SEQ ID No: 6, orra74589s02, the allele identified within SEQ ID No: 6.

In one aspect, the marker is or is linked (such as closely linked) to one or more of the following alleles:
a) allele **C**/T within SEQ ID No: 1 (ra24982s01)
b) allele C/**T** within SEQ ID No: 2 (ra74607s01)
c) allele C/**G** within SEQ ID No: 3 (ra74625s01)
d) allele C/**T** within SEQ ID No: 4 (ra74605s01)
e) allele C/**T** within SEQ ID No: 5 (ra74601s01)
f) allele **C**/T within SEQ ID No: 6 (ra74589s02)
g) allele **C**/T within SEQ ID No: 7 (ra74593s01)
h) allele G/**T** within SEQ ID No: 8 (ra25028s01)
i) allele A/**G** within SEQ ID No: 9 (ra25042s01) and/or
j) allele **G**/T within SEQ ID No: 10 (ra25063s01).
wherein the allele in bold is the allele associated with resistance to Phoma stem canker.

Thus, preferably the allele is the allele in bold above.

Suitably, a marker as described herein may detect at least one or more of the following alleles:
a) allele **C**/T of ra24982s01
b) allele C/**T** of ra74607s01
c) allele C/**G** of ra74625s01
d) allele C/**T** of ra74605s01
e) allele C/**T** of ra74601s01
f) allele **C**/T of ra74589s02
g) allele **C**/T of ra74593s01
h) allele G/**T** of ra25028s01
i) allele A/**G** of ra25042s01 and/or
j) allele **G**/T of ra25063s01.

Preferably the allele is the allele in bold above.

Suitably, a method according to the present invention may comprise detecting at least allele C/T of ra74601s01 and/or allele C/T of ra74589s02.

In one aspect, the method according to the present invention may comprise detecting more than one marker, such as at least two markers, as at least three, at least four, at least five at least six, at least seven, at least eight, at least nine at least ten markers.

Without wishing to be bound by theory, at least two markers may be used which flank *LepR1* or the haplotype as described herein. For example, at least on marker may be upstream and at least one marker may be downstream of *LepR1* or the haplotype as described herein.

**Table 1. Physical genomic positions of markers disclosed herein relative to Darmor v4.1.**

| **Marker name** | **Direction** | **Physical genomic position based on Darmor v4.1** | **Resistance allele / Sensitive allele Y** |
|---|---|---|---|
| ra24982s01 | F | 17261239 | C / T |
| ra74607s01 | R | 17643282 | T / C |
| ra74625s01 | F | 17712460 | G / C |
| ra74605s01 | F | 17869714 | T / C |
| ra74601s01* | F | 17985071 | T / C |
| ra74589s02* | F | 18087922 | C /T |
| ra74593s01 | F | 18184032 | C / T |
| ra25028s01 | F | 18255015 | T / G |
| ra25042s01 | R | 18335590 | G / A |
| ra25063s01 | F | 18698483 | G / T |

| | | | |
|---|---|---|---|
| * indicates this marker is diagnostic of the resistance haplotype | | | |

In one aspect, a method according to the present invention comprises detecting in said *B. napus* plant, or part thereof (such as tissue or seed), the presence or absence of at least two markers wherein at least one marker is on a chromosomal interval between SEQ ID No: 1 and SEQ ID No: 5, and at least one of said markers is on a chromosomal interval between SEQ ID No: 10 and SEQ ID No: 6.

In another aspect,
a) at least one of said markers is on a chromosomal interval between SEQ ID No: 2 and SEQ ID No: 5, and at least one of said markers is on a chromosomal interval between SEQ ID No: 9 and SEQ ID No: 6; and/or
b) at least one of said markers is on a chromosomal interval between SEQ ID No: 3 and SEQ ID No: 5, and at least one of said markers is on a chromosomal interval between SEQ ID No: 8 and SEQ ID No: 6; and/or
c) at least one of said markers is on a chromosomal interval between SEQ ID No: 4 and SEQ ID No: 5, and at least one of said markers is on a chromosomal interval between SEQ ID No: 7 and SEQ ID No: 6; and/or
d) at least one of said markers is on a chromosomal interval defined by SEQ ID No: 5, and at least one of said markers is on a chromosomal interval defined by SEQ ID No: 6.

On a genetic map, linkage of one molecular marker to a gene or locus or another marker is measured as a recombination frequency. A lack of precise proportionality between cM and physical distance can result in variation in recombination frequencies for different chromosomal regions for example; some regions are recombination "hotspots" whilst others have rare recombination events.

In one aspect, the at least one marker may have a recombination frequency of about 50% or less (such as about 40% or less, such as about 30% or less, such as about 20% or less, such as about 10% or less) with ra74601s01 or SEQ ID No: 5 and/or ra74589s02 or SEQ ID No: 6.

In one aspect the at least one marker may be located within 750kbp or less from ra74601s01 or SEQ ID No: 5 or ra74589s02 or SEQ ID No: 6, such as 700kbp or less, 650kbp or less, 600kbp or less, 550kbp or less, 500kbp or less, 450kbp or less, 400kbp or less, 350kbp or less, 300kbp or less, 250kbp or less, 200kbp or less, 150kbp or less, 100kbp or less, 50kbp or less, when the distance corresponds to the equivalent physical genomic positions on A02 of Darmor v4.1. Sequence alignments or contigs may also be used to find sequences upstream or downstream of the specific markers described herein. These new sequences, closely linked to the markers described herein, are then used to discover and develop functionally equivalent markers according to the present invention.

For example, different physical and/or genetic maps may be aligned to locate equivalent markers not described within this disclosure but that are within similar chromosomal regions according to the present invention.

Alignment information of the genetic and physical maps of *Brassica* and associated tools may be found at the *Brassica* Genome webpage [Hurgobin, B., et al., Plant biotechnology journal 2018, 16, 1265-1274 (2011); Nature Genetics 43: 1035-1040 Marshall et al., Plant Methods 6:19 (2010); Golicz et al., Nature Communications 7:13390 (2016)], which hosts *Brassica* genome databases and at the *Brassica* Database (BRAD) website and at the PlantGDB database .

### Commercial traits

In one aspect, the present invention relates to *B. napus* plants having resistance to Phoma stem canker and having commercially desirable traits.

In one aspect, the present invention provides a method for selecting or identifying a plant which has resistance to Phoma stem canker and which has commercially desirable traits.

Therefore, in one embodiment the methods and uses of the present invention relate to improving resistance to Phoma stem canker whilst maintaining the characteristics and/or other commercially desirable traits of a commercial *B. napus* plant (e.g. yield).

The term "commercially desirable traits" will include traits such as yield, mature plant height (for example reduced plant height may be advantageous to reduce the risk of lodging), grain grade, harvestable pod number, seed oil content, seed dry weight, number of leaves, oil quality, abiotic (for instance drought) stress tolerance, shattering resistance, herbicide tolerance and/or biotic (for instance insect, bacteria or fungus) stress tolerance.

Preferably, the yield is kernel mass. Preferably the present invention provides a method for selecting or identifying a plant which has resistance to Phoma stem canker and which has a kernel mass of at least 20dt/ha or at least 22dt/ha, preferably 24dt/ha, more preferably 26dt/ha. The kernel mass relates to the yield provided by an adult plant at the time of harvest. Commercial *B. napus* plants (plants suitable for growing commercially) typically contain lower levels or erucic acid and lower levels of glucosinolates than wild plants. Thus, the present invention provides a method for selecting or identifying a plant which has resistance to Phoma stem canker and which has low levels of eucic acid and/or glucosinolates. Preferably, the erucic acid levels are less than 2% relative to the total oil content. Preferably, the level of glucosinolates is less 25 µMol per gram of corn at a moisture content of 9%.

In one aspect, the plant has the commercially desirable traits of "canola".

"Canola" as used herein refer to an oilseed plant of the genus *Brassica* (*B. napus, Brassica rapa* or *Brassica juncea*) from which the seed oil contains less than 2% erucic acid in its fatty acid profile and the solid component contains less than 30 micromoles of any one or any mixture of 3-butenyl glucosinolate, 4-pentenyl glucosinolate, 2-hydroxy-3 butenyl glucosinolate, and 2-hydroxy- 4-pentenyl glucosinolate per gram of air-dry, oil-free solid.

In one aspect the *B. napus* plant produces an oil (after crushing the seeds) containing less than 2 % erucic acid (of the total fatty acids in the oil).

In one aspect, the solid component of the seed contains less than 30 micromoles of any one or any mixture of 3-butenyl glucosinolate, 4-pentenyl glucosinolate, 3-hydroxy-3 butenyl glucosinolate, and 2-hydroxy-4-pentenyl glucosinolate per gram of air-dry, oil-free solid.

### Breeding

Molecular markers can be used in a variety of plant breeding applications. One of the main applications is to increase the efficiency of backcrossing and introgressing genes using marker-assisted selection (MAS). A molecular marker which demonstrates linkage with a locus affecting a desired phenotypic trait provides a tool for the selection of the trait in a plant population. This may be useful where the phenotype is difficult to assay such as disease resistance traits or a trait which is measured at a late stage e.g. seed oil characteristics. Marker assays are more efficient than field phenotyping and allow much larger populations to be assayed, thereby increasing the chance of finding a recombinant with the target segment from the donor line introduced into the recipient line. The closer the linkage, the more useful the marker as recombination is less likely to occur between the marker and the gene causing the trait.

When a locus or gene is introgressed by MAS, it is not only the gene that is introduced but also the flanking regions. This is typically referred to as "linkage drag." These flanking regions can carry additional genes that may encode commercially or agronomically undesirable traits. This "linkage drag" may also result in reduced yield or other negative agronomic characteristics even after multiple cycles of backcrossing into the elite line. This is also sometimes referred to as "yield drag." The size of the flanking region can be decreased by additional backcrossing, although this is not always successful, as breeders do not have control over the size of the region or the recombination. In classical breeding it is usually only by chance that recombinations are selected that contribute to a reduction in the size of the donor segment. With markers however, it is possible to select rare individuals that have experienced recombination near the target region (such as the *LepR1* locus). When the location of the target region is known, a series of flanking markers surrounding the target as described herein can be used to select for recombinations in different population sizes.

The key steps for MAS are: (i) Defining the population within which the marker-trait association will be determined, which can be a segregating population, or a random or structured population; (ii) monitoring the segregation or association of polymorphic markers relative to the trait, and determining linkage or association using statistical methods; (iii) defining a set of desirable markers based on the results of the statistical analysis, and (iv) the use and/or extrapolation of this information to the current set of breeding germplasm to enable marker-based selection decisions to be made. Different types of markers can be used in marker assisted selection protocols for example: SNP, SSR and FLP markers.

In one aspect, the present invention provides markers for MAS of *B. napus* plants having resistance to Phoma stem canker. In another aspect, the present invention provides kits of primers for MAS of *B. napus* plants having resistance to Phoma stem canker.

Other aspects of the present invention include methods for identifying *B. napus* plants which have newly conferred or enhanced resistance to Phoma stem canker by detecting markers which have been introgressed into said plant.

In another aspect the present invention provides a method for selecting or identifying an introgressed plant having resistance to Phoma stem canker and which has commercially desirable traits.

Suitably, the markers according to the present invention may be used to select or identify introgressed plants having resistance to Phoma stem canker whilst reducing or preventing any negative agronomic characteristics which may be caused by linkage drag.

The method according to the present invention may be used to select or identify a *B. napus* plant having resistance to Phoma stem canker, wherein said *B. napus* plant has been obtained by a process of introgressing *LepR1* from a donor plant into a recipient *B. napus* plant to produce an introgressed *B. napus* plant.

The process of introgressing is also referred to as "backcrossing" when the process is repeated two or more times.

A "donor plant" as used herein refers to a plant which comprises at least one of the markers described herein and which has resistance to Phoma stem canker. The donor plant comprises the desired gene or locus to be introgressed into a recipient plant.

A "recipient plant" as used herein refers to the plant into which the gene or locus is being introgressed.

The initial cross gives rise to the F1 generation; the term "BC1" then refers to the second use

of the recipient or recurrent parent, "BC2" refers to the third use of the recurrent parent, and so on.

Suitably, the method according to the present invention may reduce or eliminate linkage drag from the donor plant and may lead to a agronomically useful line or variety or to a plant which can be used in a breeding program (for example a DH line).

Suitably, the donor plant may be *Brassica rapa* subsp. *sylvestris (AA genome).*

The recipient plant may be any *B. napus* plant for which it is desired to increased resistance to Phoma stem canker.

In one aspect, the recipient plant may be an elite line or elite variety.

As used herein, an "elite line" or "elite variety" is an agronomically superior line or variety that has resulted from many cycles of breeding and selection for superior agronomic performance. An "elite inbred line" is an elite line that is an inbred, and that has been shown to be useful for producing sufficiently high yielding and agronomically fit hybrid varieties (an "elite hybrid variety"). Numerous elite lines and varieties are available and known to those of skill in the art of *Brassica* breeding. Similarly, "elite germplasm" is an agronomically superior germplasm, typically derived from and/or capable of giving rise to a plant with superior agronomic performance, such as an existing or newly developed elite line of *Brassica.*

Included herein is a method for farming of plants of the genus Brassica or the species Brassica napus, including
(i) the provision of a resistant plant or seed by one of the methods for selection or identification described herein
(ii) the planting of the plant or seed obtained by step (i) and
(iii) cultivation of the plant or seed,
wherein the method counteracts an infestation of the cultivated plants with Leptosphaeria maculans and/or L. biglobosa. The planting in step (ii) may occur in an area infested by Leptosphaeria maculans and/or L. biglobosa. In a preferred embodiment the method is further characterized in that the amount of applied fungicides is reduced in comparison to the planting of plant or seed not comprising the resistance according to the invention. As fungal spores may survive on a agricultural field or within the soil over a long period in a further preferred embodiment of the above given method the method is repeated for 2, 3, 4 or even 5 years within the same area (for example on the same field). The cultivation in step (iii) should occur under conditions which allow a plant to grow and / or a seed to germinate and subsequently grow. That means that the plant or the seed should be brought into a suitable substrate (like for example soil) wherein the substrate contains sufficient amount of water (like 10 - 35% g/g) and wherein a suitable light source (for example sunshine) triggers photosynthesis during at least a part of the day (for example 8 - 18h / d). In a preferred embodiment of the method the plant or seed provided by step (i) is homozygous for the resistance. The method and every embodiment of it may optionally include a step (iv) harvesting of the plant or plant material like for example seeds. The farming method given above may be part of a method for producing canola oil. The method for production canola oil includes the steps (i) - (iv), and additionally step (v) the extracting of oil from the seeds harvested in step (iv).

### Plants

As used herein "plant or part thereof" includes a plant or any part thereof, such plant tissue, roots, leaves, stem, infloresence, immature pods, pod walls and seeds.

In Europe, winter-type cultivars are grown as an annual break crop in rotation with cereals and break crops. Winter-type cultivars are typically more vigorous than summer varieties and less susceptible to crop failure.

"Winter" or "winter-type" as used herein refers to crops which require vernalization to initiate the flowering process.

Examples of winter-type cultivars include Darmor-bzh.

"Spring" or "spring-type" as used herein refers to crops do not require vernalization to initiate the flowering process.

In Canada and Australia spring cultivars which are not winter-hardy and do not require vernalization are grown.

In one aspect, the *B. napus* plant is an introgressed or backcrossed plant.

In one aspect, the donor plant is a spring-type cultivar.

In one aspect, the recipient plant is a winter-type cultivar.

For example, the markers described herein may be used to introduce Phoma stem canker resistance (*LepR1* on chromosome A02) from a spring cultivar into a winter-type cultivar. The markers described herein can be used to minimize linkage drag of the spring source from chromosome A02.

### KIT

The present invention also relates to a kit of primers for identifying a *B. napus* plant having resistance to Phoma stem canker (such as resistance to fungal pathogen(s) *L. maculans* and/or *L. biglobosa).*

Suitably, the kit may comprise primers capable of identifying the presence or absence of at least one of the markers described herein.

Suitably, the kit may comprise primers capable of identifying the presence or absence of at least one of the following markers:
a) ra24982s01; and/or
b) ra74607s01; and/or
c) ra74625s01; and/or
d) ra74605s01; and/or
e) ra74601s01; and/or
f) ra74589s02; and/or
g) ra74593s01; and/or
h) ra25028s01; and/or
i) ra25042s01; and/or
j) ra25063s01.

Suitably, the kit of primers may comprise at least two, at least three, at least four, at least five, at least six, at least seven, at least eight, at least nine, at least ten sets of primers as defined in a) to j) above.

In one aspect, the kit of primers is capable of identifying the presence or absence of at least ra74601s01.

In one aspect, the kit of primers is capable of identifying the presence or absence of at least ra74589s02.

Suitably, the kit of primers may comprise primers capable of identifying the presence or absence of at least one of the following pairs of markers:
a) ra24982s01 and ra25063s01;
b) ra74607s01 and ra25042s01;
c) ra74625s01 and ra25028s01;
d) ra74605s01 and ra74593s01;
e) ra74601s01 and ra74589s02.

Suitably, the kit of primers may comprise at least two, at least three, at least four or all five sets

of primers as defined in a) to e) above.

In one aspect, the kit of primers is capable of identifying the presence or absence of at least ra74601s01 and ra74589s02.

In one aspect, the present invention provides a kit of primers for identifying a *B. napus* plant having resistance to Phoma stem canker (such as resistance to fungal pathogen(s) *L. maculans* and/or *L. biglobosa)* comprising at least one of:
a) a set of primers capable of identifying the presence or absence of marker ra24982s01, comprising a primer comprising SEQ ID No: 11 or a primer comprising SEQ ID No: 12 and a primer comprising SEQ ID No. 13; and/or
b) a set of primers capable of identifying the presence or absence of marker ra74607s01, comprising a primer comprising SEQ ID No: 14 or a primer comprising SEQ ID No: 15 and a primer comprising SEQ ID No. 16; and/or
c) a set of primers capable of identifying the presence or absence of marker ra74625s01, comprising a primer comprising SEQ ID No: 17 or a primer comprising SEQ ID No: 18 and a primer comprising SEQ ID No. 19; and/or
d) a set of primers capable of identifying the presence or absence of marker ra74605s01, comprising a primer comprising SEQ ID No: 20 or a primer comprising SEQ ID No: 21 and a primer comprising SEQ ID No. 22; and/or
e) a set of primers capable of identifying the presence or absence of marker ra74601s01, comprising a primer comprising SEQ ID No: 23 or a primer comprising SEQ ID No: 24 and a primer comprising SEQ ID No. 25; and/or
f) a set of primers capable of identifying the presence or absence of marker ra74589s02, comprising a primer comprising SEQ ID No: 26 or a primer comprising SEQ ID No: 27 and a primer comprising SEQ ID No. 28; and/or
g) a set of primers capable of identifying the presence or absence of marker ra74593s01, comprising a primer comprising SEQ ID No: 29 or a primer comprising SEQ ID No: 30 and a primer comprising SEQ ID No. 31; and/or
h) a set of primers capable of identifying the presence or absence of marker ra25028s01, comprising a primer comprising SEQ ID No: 32 or a primer comprising SEQ ID No: 33 and a primer comprising SEQ ID No. 34; and/or
i) a set of primers capable of identifying the presence or absence of marker ra25042s01, comprising a primer comprising SEQ ID No: 35 or a primer comprising SEQ ID No: 36 and a primer comprising SEQ ID No. 37; and/or
j) a set of primers capable of identifying the presence or absence of marker ra25063s01, comprising a primer comprising SEQ ID No: 38 or a primer comprising SEQ ID No: 39 and a primer comprising SEQ ID No. 40.

Suitably, the kit of primers may comprise at least two, at least three, at least four, at least five, at least six, at least seven, at least eight, at least nine, at least ten sets of primers as defined in a) to j) above.

In one aspect, the kit of primers is for identifying a *B. napus* plant having resistance to resistance to Phoma stem canker and which have commercially desirable traits. The invention furthermore relates to a pair of primers as mentioned in context of the kit. Preferably a pair of those primers is a pair as defined in a) to j) above. A primer may be an oligonucleotide and / or a marker preferably a molecular marker.

### USE

In one aspect, the present invention provides the use of at least one marker as described herein, or a sequence selected from SEQ ID Nos: 1-10, or a kit of primers as described herein, to select a *B. napus* plant having resistance to Phoma stem canker (such as resistance to fungal pathogen(s) *L*. *maculans and*/*or L. biglobosa).*

Suitably, the method may comprise selecting a *B. napus* plant having a marker associated with resistance to Phoma stem canker (such as fungal pathogen(s) *L. maculans and*/*or L. biglobosa)* as described herein.

In one aspect, the present invention relates to the use of at least one marker described herein, or at least one sequence selected from SEQ ID Nos: 1-10 or a kit of primers according to the present invention, to select a *B. napus* plant having resistance to Phoma stem canker (such as resistance to fungal pathogen(s) *L. maculansand*/*or L. biglobosa).*

Suitably, the use may comprise the use of at least two, at least three, at least four, at least five, at least six, at least seven, at least eight, at least nine, or ten markers as described herein, (such as the markers SEQ ID Nos: 1-10) to select a *B. napus* plant having resistance to Phoma stem cankcer (such as fungal pathogen(s) *L. maculans and*/*or L. biglobosa).*

In one aspect, a use according to the present invention comprises the use of at least ra74601s01 or SEQ ID No: 5.

In one aspect, a use according to the present invention comprises the use of at least ra74589s02 or SEQ ID No: 6.

In one aspect, a use according to the present invention comprises the use of at least ra74601s01 or SEQ ID No: 5 and ra74589s02 or SEQ ID No: 6.

The present invention also provides the use of a plant selected by a method according to the present invention, for producing an oilseed rape oil or an oilseed rape seed cake.

In one aspect, a method for producing an oilseed rape oil or an oilseed rape seed cake comprises detecting in said *B. napus* plant, or part thereof (such as tissue or seed), the presence or absence of at least one marker as described herein (for example which is on a chromosomal interval between SEQ ID No: 1 and SEQ ID No: 10.

Suitably, a method or use according to the present invention may comprise extraction of DNA from plant tissue and carrying analysing said DNA for the presence or absence of one or more markers as defined herein.

Suitably, a method or use according to the present invention may comprise growing a plant or plants identified as having a marker associated with resistance to Phoma stem cankcer (such as fungal pathogen(s) *L. maculans and*/*or L. biglobosa).*

Suitably, a method or use according to the present invention may comprise processing of rapeseed for oil production.

Suitably, a method or use according to the present invention may comprise the production of rapeseed meal.

It is thought that erucic acid and glucosinolates may be disadvantageous in animal feed. For example canola oil is limited by government regulation to a maximum of 2% erucic acid by weight in the USA and 5% in the EU.

As described herein, plants selected by methods of the present invention may comprise lower levels of erucic acid and lower levels of glucosinolates compared with other *B. napus* plants having resistance to fungal pathogen(s) *L. maculans and*/*or L. biglobosa,* such as wild *Brassica* plants. Therefore *B. napus* plants produced by methods according to the present invention may have higher nutritional value of rapeseed press cakes for animal feed.

Suitably, a method or use according to the present invention may comprise the production of a plant that produces an oil, after crushing the seeds, containing less than 5 % (such as less than 4%, such as less than 3%, such as less than 2%, such as less than 1%) erucic acid of the total fatty acids in the oil.

Suitably, a method or use according to the present invention may comprise the production of a plant that produces an oil, after crushing the seeds, containing less than 5 % (such as less than 4%, such as less than 3%, such as less than 2%, such as less than 1%) erucic acid of the total fatty acids in the oil.

Suitably, a method or use according to the present invention may comprise the production of a plant that contains a level of aliphatic glucosinolates in dry, defatted seed meal of less than 30 micromol/g

### EXAMPLES

### Example 1 - Development of an introgression line of elite breeding material harbouring the resistant allele/haplotype of the Blackleg resistance locus LepR1 and a diagnostic marker set for selection of the resistant allele/haplotype

The monogenic Blackleg resistance locus *LepR1* has been described previously. A rough genetic map consisting of 4 SSRs based on chromosome A02 was established (see Figure 1). The size of the *LepR1* target region was at that time, based on the public genomic reference Darmor v4.1, about 6.5Mb.

The present inventors sought to develop an introgression line of elite breeding material harbouring the resistant allele/haplotype of the Blackleg resistance locus *LepR1* and a diagnostic marker set for selection of the resistant allele/haplotype.

The BC2S1 (backcrossing generation 2, first selfing) (09T11R1-13) was the basis for a backcrossing program integrating the Blackleg resistance gene into elite winter restorer material, whilst trying in parallel to minimize the linkage drag of the spring source on chromosome A02.

The 20 received seeds of 09T11R1-13, still segregating for the resistance trait, were in the first step selfed in the greenhouse to multiply the material. The resulting seeds were checked in a cotyledon test for Blackleg resistance with isolate T12aD34, a typical German Blackleg field isolate. For the test, single plants were inoculated on their two cotyledons with two drops (one each half of cotyledon) of an inoculum of race T12aD34. After about 10 days the symptoms were scored on a scale of 1-6 (1-3 = resistant, 4-6 = susceptible).

In this resistance test, the progeny of a single self could be fully resistant (selfed plant homozygous for *LepR1),* fully susceptible (selfed plant non-carrying *LepR1)* or segregating for resistance (selfed plant heterozygous *LepR1).* Plants showing a resistance score of 1 (fully resistant no disease symptoms) were selected and crossed to KWS elite restorer lines.

In the resulting BC0 the plants could all be heterozygous (donor plant homozygous *LepR1)* or segregating for heterozygous versus non-carrying (donor plant heterozygote *LepR1)* plants.

Due to the fact that the *LepR1* resistance is dominant, a plant showing resistance could be homozygote or heterozygote carrying the resistance gene. If BC0 would be generated just by chance with a plant coming from that test, the next generation could be fully heterozygote (selected plant = homozygote for *LepR1)* or segregating (selected plant = heterozygote). Therefore, plant material was again tested in the cotyledon test and resistant heterozygous plants selected to generate BC1 (backcrossing generation 1). From BC2 to BC4 (backcrossing generation 2 and 4) cotyledon test was repeated selecting for resistant heterozygous plants. After BC4 the material went into the forward breeding program, for development of DH (double haploid) lines and testing their performance in intensive GCA- (general combining ability) and SCA- (special combining ability) trials.

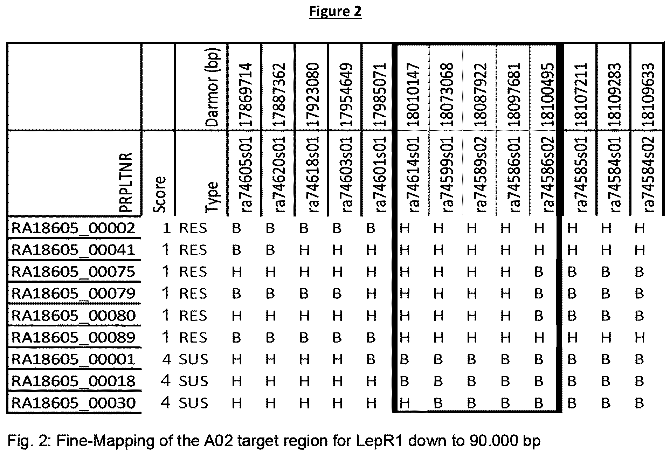

In BC2S1 (in 2012) a first internal genetic mapping approach was performed confirming the A02 resistance locus. In the following years further genetic- and QTL-mapping analyses in different BC-/BCS-/DH generations (DH= double haploid) preformed by the inventors brought down the target region to 90kbp based on physical mapping of markers on reference genome Darmor v4.1 (see Figure 2).

This target region now comprises 21 genes in the Darmor reference. The reduction in target size was based on improvements in map marker density and screening for recombinants. In the first two years few new SSR makers were placed, followed by markers selected from public i60k Illumina CHIP converted RD-LS-MS to KASPAR markers.

The latest marker developments started based on Illumina Xten resequencing data of a DH line carrying the *LepR1* target region. Today a total set of 115 KASPAR and 33 XT-CHIP markers (28 overlapping) are used for analysis of the target region including flanking regions (4MB, 16-20 Mio bp on physical map Darmor v4.1). With two markers (ra74589s02 and ra74601s01) the present inventors identified a diagnostic haplotype spanning a region of about 110kbp. *Example 2* - *LepR1* lines show improved resistance scores compared to susceptible lines and lines carrying only quantitative resistance.

In order to show the value of the new resistance locus for oilseed rape breeding, different field trials were analysed. A subset of DH lines with the following resistance: *LepR1, LepR2* and *Rlm3* were tested in field trial O18-RA W/PH1 with PG2 score.

For the scoring about 20 plants per genotype were cut at the root base and the percentage of infected root tissue was estimated with a score from 1-9 as the basis for the disease index PG2. The summary of the results is given in Table 2, below showing a clear improvement in PG2 score of *LepR1* carrying DH lines compared to susceptible lines such as Capitol.

**Table 2: PG2 scoring trial O18-RAW/PH1**

| **Genotype** | **No of lines** | **Average PG2 score** |
|---|---|---|
| Capitol | 3 | 2,76 |
| Express | 3 | 0,98 |
| Exquisite | 3 | 0,88 |
| Rlm3 carrying DH | 127 | 0,89 |
| LepR2 carrying DH line | 47 | 0,52 |
| LepR1 carrying DH line | 86 | 0,51 |

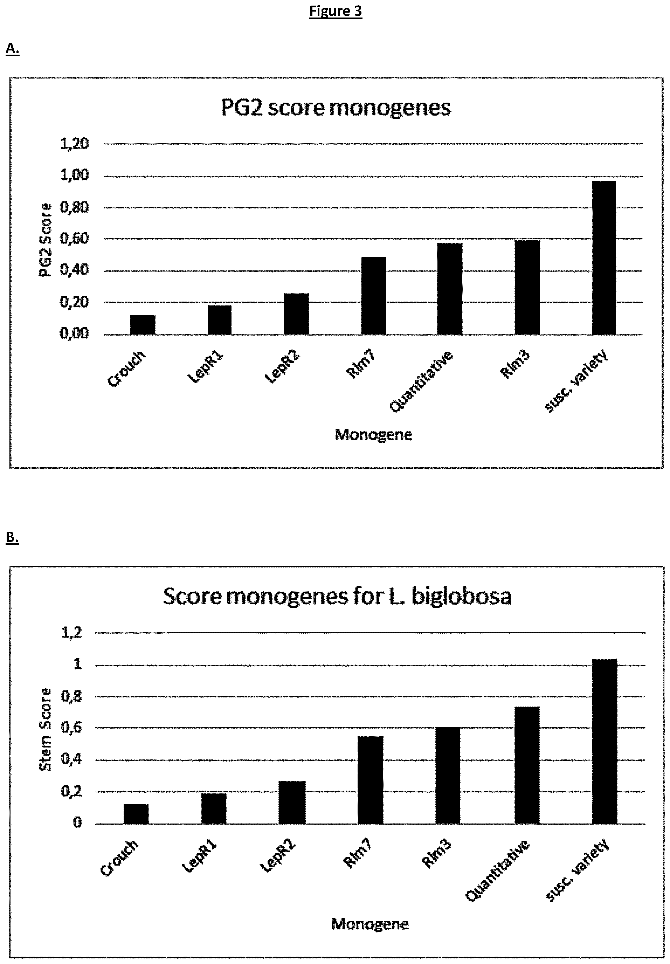

Furthermore, DH lines carrying different monogenic resistance genes were tested over three years for *L. maculans* and *L. biglobosa* resistance (see Figure 3A and Figure 3B).

*LepR1* shows better resistance scores for both pathogens compared to the susceptible lines and lines carrying only quantitative resistance. The disease level in *LepR1* lines is also relatively low compared to *Rlm7-* (partially broken) or *Rlm3*-lines.

Using internally the "Fixed Effect" approach in genomic prediction, the effect of a monogenic versus quantitative resistance can be estimated. For fixed effects, it is typically required that a marker is linked as closely as possible to the trait.

This was performed for breeding material in 2018. The trait PMR was used, where Blackleg resistance is one of the main components. PMR is mainly driven by diseases and in European fields, a main driver is Blackleg. Therefore it was expected that lower PMR values would be correlated with *LepR1.*

The intercept for PMR in the breeding program was 5.8. DH lines carrying the *LepR1* resistance show a decrease in score by 0.68. Therefore, lines including *LepR1* resistance can be used as Blackleg resistance varieties and used commercially as agricultural lines.

In comparison, *Rlm7* lines lower the intercept by only 0.2, showing that the resistance is not strong anymore, as first breakage is seen in Europe. Further testing performed in 2019 produce similar results.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, cellular immunology or related fields are intended to be within the scope of the following claims.

## Claims

1. A method for selecting or identifying a *Brassica napus* plant having resistance to fungal pathogen(s) *Leptosphaeria maculans and*/*or L. biglobosa,* comprising detecting in said *Brassica napus* plant, or part thereof, the presence or absence of at least one marker which is on or within a chromosomal interval between ra24982s01 according to SEQ ID No: 1 and ra25063s01 according to SEQ ID No: 10.

2. The method according to claim 1, wherein said at least one marker is on or within a chromosomal interval between ra74607s01 according to SEQ ID No: 2 and ra25042s01 according to SEQ ID No: 9.

3. The method according to claim 1 or claim 2, wherein said at least one marker is on or within a chromosomal interval between ra74625s01 according to SEQ ID No: 3 and ra25028s01 according to SEQ ID No: 8.

4. The method according to any one of claims 1 to 3, wherein said at least one marker is on or within a chromosomal interval between ra74605s01 according to SEQ ID No: 4 and ra74593s01 according to SEQ ID No: 7.

5. The method according to any one of claims 1 to 4, wherein said at least one marker is on or within a chromosomal interval between ra74601s01 according to SEQ ID No: 5 and ra74589s02 according to SEQ ID No: 6.

6. The method according to any one of claims 1 to 5, wherein the method comprises detecting at least one or more of the following alleles:
a) allele y or allele c at ra24982s01 according to SEQ ID No: 1
b) allele y or allele t at ra74607s01 according to SEQ ID No: 2
c) allele s or allele g at ra74625s01 according to SEQ ID No: 3
d) allele y or allele t at ra74605s01 according to SEQ ID No: 4
e) allele y or allele t at ra74601s01 according to SEQ ID No: 5
f) allele y or allele c at ra74589s02 according to SEQ ID No: 6
g) allele y or allele c at ra74593s01 according to SEQ ID No: 7
h) allele k or allele t at ra25028s01 according to SEQ ID No: 8
i) allele r or allele g at ra25042s01 according to SEQ ID No: 9
j) allele k or allele g at ra25063s01 according to SEQ ID No: 10.

7. The method according to any preceding claim, wherein said method comprises detecting in said *Brassica napus* plant, or part thereof (such as tissue or seed), the presence or absence of at least two markers wherein at least one marker is on or within a chromosomal interval between ra24982s01 according to SEQ ID No: 1 and ra74601s01 according to SEQ ID No: 5, and at least one of said markers is on or within a chromosomal interval between ra25063s01 according to SEQ ID No: 10) and ra74589s02 according to SEQ ID No: 6.

8. The method according to claim 7, wherein:
a) at least one of said markers is on or within a chromosomal interval between ra74607s01 according to SEQ ID No: 2 and ra74601s01 according to SEQ ID No: 5, and at least one of said markers is on or within a chromosomal interval between ra25042s01 according to SEQ ID No: 9 and ra74589s02 according to SEQ ID No: 6; and/or
b) at least one of said markers is on or within a chromosomal interval between ra74625s01 according to SEQ ID No: 3 and ra74601s01 according to SEQ ID No: 5, and at least one of said markers is on or within a chromosomal interval between ra25028s01 according to SEQ ID No: 8 and ra74589s02 according to SEQ ID No: 6; and/or
c) at least one of said markers is on or within a chromosomal interval between ra74605s01 according to SEQ ID No: 4 and ra74601s01 according to SEQ ID No: 5, and at least one of said markers is on or within a chromosomal interval between ra74593s01 according to SEQ ID No: 7 and ra74589s02 according to SEQ ID No: 6; and/or
d) at least one of said markers is on or within a chromosomal interval defined by ra74601s01 according to SEQ ID No: 5, and at least one of said markers is on or within a chromosomal interval defined by ra74589s02 according to SEQ ID No: 6.

9. The method according to any preceding claim, wherein the *Brassica napus* plant has an AACC genome and has been obtained by a process of introgressing LepR1 from a donor plant into a recipient *Brassica napus* plant to produce an introgressed *Brassica napus* plant.

10. The method according to claim 9, wherein the donor plant is *Brassica rapa* subsp. *sylvestris having a AA genome.*

11. A method according to claim 10, wherein the introgressed *Brassica* plant is selected for a recombination event on or within a chromosomal interval between ra24982s01 according to SEQ ID No: 1 and ra25063s01 according to SEQ ID No: 10 and does not retain a second chromosomal interval derived from the donor plant.

12. A method according to any one of claims 1 to 11, wherein said *Brassica napus* plant or part thereof does not exhibit any negative agronomic and/or phenotypic properties associated with

13. A pair of primers for identifying a *Brassica napus* plant having resistance to fungal pathogen(s) *Leptosphaeria maculans* and/or *L*. *biglobosa* wherein the pair of primers is selected from the group consisting of:
a) a pair of primers comprising SEQ ID No: 11 and SEQ ID No: 12; and/or
b) a pair of primers comprising SEQ ID No: 13 and SEQ ID No: 14; and/or
c) a pair of primers comprising SEQ ID No: 15 and SEQ ID No: 16; and/or
d) a pair of primers comprising SEQ ID No: 17 and SEQ ID No: 18; and/or
e) a pair of primers comprising SEQ ID No: 19 and SEQ ID No: 20; and/or
f) a pair of primers comprising SEQ ID No: 21 and SEQ ID No: 22; and/or
g) a pair of primers comprising SEQ ID No: 23 and SEQ ID No: 24; and/or
h) a pair of primers comprising SEQ ID No: 25 and SEQ ID No: 26; and/or
i) a pair of primers comprising SEQ ID No: 27 and SEQ ID No: 28; and/or
j) a pair of primers comprising SEQ ID No: 29 and SEQ ID No: 30.

14. Use of a plant selected by any one of claims 1 to 12, for producing an oilseed rape oil or an oilseed rape seed cake.

15. Use of at least one marker selected from SEQ ID No: 1-10 or a par of primers according to claim 13, to select a *Brassica napus* plant having resistance to fungal pathogen(s) *Leptosphaeria maculans and*/*or L. biglobosa.*
